(19) Europäisches Patentamt
European Patent Office
Office européen des brevets

(11) EP 0 835 254 B1

(12) **FASCICULE DE BREVET EUROPEEN**

(45) Date de publication et mention de la délivrance du brevet:
**01.09.1999 Bulletin 1999/35**

(21) Numéro de dépôt: **96918719.4**

(22) Date de dépôt: **28.05.1996**

(51) Int Cl.$^6$: **C07D 413/04**, C07D 417/04, A61K 31/42, A61K 31/425

(86) Numéro de dépôt international:
**PCT/FR96/00792**

(87) Numéro de publication internationale:
**WO 96/38444 (05.12.1996 Gazette 1996/53)**

(54) **DERIVES D'OXAZOLIDINONE, LEUR PREPARATION ET LEUR APPLICATION EN THERAPEUTIQUE**

OXAZOLIDINONE DERIVATE, IHRE HERSTELLUNG UND IHRE THERAPEUTISCHE VERWENDUNG

OXAZOLIDINONE DERIVATIVES, THEIR PREPARATION AND THERAPEUTICAL USE

(84) Etats contractants désignés:
**AT BE CH DE DK ES FI FR GB GR IE IT LI LU NL PT SE**

(30) Priorité: **02.06.1995 FR 9506563**
**02.06.1995 FR 9506564**

(43) Date de publication de la demande:
**15.04.1998 Bulletin 1998/16**

(73) Titulaire: **SYNTHELABO**
**92350 Le Plessis Robinson (FR)**

(72) Inventeurs:
- **JEGHAM, Samir**
  **F-95100 Argenteuil (FR)**
- **PUECH, Frédéric**
  **F-92500 Rueil-Malmaison (FR)**
- **BURNIER, Philippe**
  **F-78600 Maisons-Laffitte (FR)**

(74) Mandataire: **Thouret-Lemaitre, Elisabeth et al**
**Sanofi-Synthélabo**
**Service Brevets**
**174, avenue de France**
**75013 Paris (FR)**

(56) Documents cités:
**EP-A- 0 425 209** **EP-A- 0 428 421**
**EP-A- 0 494 817**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet des dérivés d'isoindoles, plus particulièrement des dérivés de 5-(hydroxyméthyl)oxazolidin-2-one, substitués en 3- par un noyau indazole, benzisoxazole, ou benzisothiazole, leur procédé de préparation et leur application en thérapeutique.

On connaît de la demande de brevet EP-A-0 425 209 des dérivés de la naphtyloxazolidone, actifs comme inhibiteurs de la monoamine oxydase (MAO).

La présente invention a pour objet de nouveaux composés répondant à la formule générale (I)

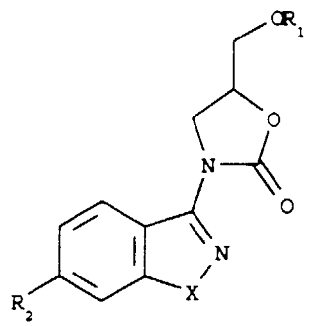

(I)

dans laquelle :

X représente un atome d'oxygène, un atome de soufre, ou un groupe NR, R étant un atome d'hydrogène, ou une chaîne alkyle en $C_1$-$C_4$, linéaire ou ramifiée,
$R_1$ représente un atome d'hydrogène ou un groupe méthyle, et
$R_2$ représente :

(i) un groupe $R_3O$ dans lequel $R_3$ représente soit un atome d'hydrogène, soit un groupe benzyle éventuellement substitué par un atome d'halogène ou par un groupe nitro ou méthylènedioxy, soit un groupe méthoxyéthyle, butyle, 4,4,4-trifluorobutyle, 4,4,4-trifluoro-3-hydroxybutyle ou 4,4,4-trifluorobut-2-ényle,
ou (ii) un groupe -CH=CH-$R_4$ ou -$CH_2$-$CH_2$-R, et dans lequel $R_4$ représente un atome d'hydrogène ou un groupe phényle, 3,3,3-trifluoropropyle ou 3,3,3-trifluoro-2-hydroxypropyle.

Les composés de formule (I) comportent un ou deux atomes de carbone asymétriques. Ils peuvent donc exister sous forme d'énantiomères ou de diastéréoisomères. Ces différentes formes ainsi que leurs mélanges, y compris les mélanges racémiques, font partie de l'invention.

Les composés de formule (I) dans laquelle X est un atome d'oxygène et $R_2$ représente un groupe -CH=CH-$R_4$, à l'exception des composés dans lesquels $R_4$ est un atome d'hydrogène, existent sous forme d'isomères cis ou trans. Ces formes, ainsi que leurs mélanges, font partie de l'invention.

Les composés de formule (I) dans laquelle $R_2$ représente un groupe $OR_3$ dans lequel $R_3$ représente le groupe 4,4,4-trifluorobut-2-ényle, existent sous forme d'isomères cis ou trans. Ces formes, ainsi que leurs mélanges, font partie de l'invention.

Les composés préférés sont ceux pour lesquels :

X représente un atome d'oxygène, un atome de soufre, ou un groupe NR, R étant un atome d'hydrogène, ou une chaîne alkyle en $C_1$-$C_4$, linéaire ou ramifiée,
$R_1$ représente un groupe méthyle ou un atome d'hydrogène, et
$R_2$ représente un groupe $R_3O$ dans lequel $R_3$ représente soit un atome d'hydrogène, soit un groupe benzyle éventuellement substitué par un atome d'halogène ou par un groupe nitro ou méthylènedioxy, soit un groupe méthoxyéthyle, butyle, 4,4,4-trifluorobutyle, 4,4,4-trifluoro-3-hydroxybutyle ou 4,4,4-trifluorobut-2-ényle,

sous forme d'énantiomères ou de diastéréoisomères, ou de mélanges de ces différentes formes, y compris de mélanges racémiques.

D'autres composés préférés sont ceux pour lesquels :

X représente un atome d'oxygène, un atome de soufre, ou un groupe NR, R étant un atome d'hydrogène, ou une

chaîne alkyle en $C_1$-$C_4$, linéaire ou ramifiée,
$R_1$ représente un groupe méthyle ou un atome d'hydrogène, et
$R_2$ représente un groupe -CH=CH-$R_4$ ou -$CH_2$-$CH_2$-$R_4$ et dans lequel $R_4$ représente un atome d'hydrogène ou un groupe phényle, 3,3,3-trifluoropropyle ou 3,3,3-trifluoro-2-hydroxypropyle,

sous forme d'énantiomères ou de diastéréoisomères, ou de mélanges de ces différentes formes, y compris de mélanges racémiques.

Les composés de choix sont ceux pour lesquels :

X représente un atome d'oxygène,
$R_1$ représente un groupe méthyle ou un atome d'hydrogène, et
$R_2$ représente :

(i) un groupe $R_3O$ dans lequel $R_3$ représente soit un atome d'hydrogène, soit un groupe benzyle, éventuellement substitué par un atome d'halogène ou par un groupe nitro ou méthylènedioxy, soit un groupe méthoxyéthyle, butyle, 4,4,4-trifluorobutyle, 4,4,4-trifluoro-3-hydroxybutyle ou 4,4,4-trifluorobut-2-ényle,
ou (ii) un groupe -CH=CH-$R_4$ ou -$CH_2$-$CH_2$-$R_4$ et dans lequel $R_4$ représente un atome d'hydrogène ou un groupe phényle, 3,3,3-trifluoropropyle ou 3,3,3-trifluoro-2-hydroxypropyle, sous forme d'énantiomères ou de diastéréoisomères, ou de mélanges de ces différentes formes, y compris de mélanges racémiques.

Parmi ceux-ci, on peut citer les composés pour lesquels :

X représente un atome d'oxygène,
$R_1$ représente un groupe méthyle ou un atome d'hydrogène, et
$R_2$ représente soit un groupe hydroxyle, soit un groupe phénylméthoxyle, éventuellement substitué par un atome d'halogène ou par un groupe nitro ou méthylènedioxy, soit un groupe 4,4,4-trifluorobutoxyle, soit un groupe 4,4,4-trifluoro-3-hydroxybutoxyle,

sous forme d'énantiomères ou de diastéréoisomères, ou de mélanges de ces différentes formes, y compris de mélanges racémiques,
et tout particulièrement, on peut citer la (*S*)-5-méthoxyméthyl-3-[6-(4,4,4-trifluorobutoxy)-1,2-benzisoxazol-3-yl]oxazolidin-2-one.

Les composés de formule (I) dans laquelle $R_1$ est un groupe méthyle, et $R_2$ est un groupe $OR_3$, peuvent être préparés selon le procédé représenté dans l'annexe 1. Selon ce procédé,on traite le dérivé d'isoindole de formule (II) par l'un des isomères 4*(R)* ou 4*(S)* de la 4-méthoxyméthyl-1,3-dioxolan-2-one de formule (IIIa), en présence de carbonate de potassium, pour obtenir l'isomère 5*(S)* ou 5*(R)* du composé de formule (Ia) selon l'invention. On peut ensuite débenzyler le composé (Ia) par hydrogénation catalytique, dans des conditions classiques pour l'homme du métier, ou à l'aide d'un acide de Lewis tel que le chlorure d'aluminium, pour obtenir l'isomère 5(*S*) ou 5*(R)* du composé de formule (Ib) selon l'invention. Puis on peut traiter le composé (Ib) soit par un composé de formule $R_3Y$ dans laquelle $R_3$ est défini comme dans la formule (I) ci-dessus, à l'exception des valeurs hydrogène et benzyle non substitué, et Y est un groupement partant, tel qu'un atome de chlore ou de brome ou un groupe tosyloxy, en présence de carbonate de potassium, soit par un composé de formule $R_3OH$ dans laquelle $R_3$ est défini comme précédemment, en présence de triphénylphosphine et d'azodicarboxylate de diéthyle, pour obtenir les isomères 5(*S*) ou 5(*R*) des composés de formule (Ic) selon l'invention, dans laquelle $R_3$ est défini comme précédemment. Dans chacun des composés de formule (II), (Ia) , (Ib), (Ic) mentionnés ci-desssus, X a l'une des significations données dans la formule (I), et Bn représente le groupement benzyle.
Les composés de formule (I) pour lesquels X représente un groupe NH, peuvent être préparés d'après les composés de formule (Ic), où X représente un groupe $NCH_3$, par déméthylation à l'aide de peroxyde de benzoyle.

Les composés de formule (I) dans laquelle $R_1$ est un groupement méthyle, et $R_2$ est un groupe -CH=CH-$R_4$ ou un groupe -$CH_2$-$CH_2$-$R_4$, peuvent être préparés selon le procédé représenté dans l'annexe 2. Selon ce procédé, on traite le composé de formule (Ib) selon l'invention par l'anhydride trifluorométhanesulfonique. On fait réagir le composé de formule (IV) ainsi obtenu, avec le tributylvinylétain en présence de chlorure de lithium et de tétrakis(triphénylphosphine)palladium. On traite alors le composé de formule (Id) selon l'invention par l'ozone, puis par le diméthylsulfure, dans le dichlorométhane, et on fait réagir le composé de formule (V) obtenu avec un iodure de triphénylphosphonium de formule $R_4CH_2PPh_3^+I^-$ dans laquelle $R_4$ est défini comme dans la formule (I) ci-dessus, à l'exception de la valeur hydrogène, en présence de carbonate de potassium. On réduit alors le composé de formule (Ie) selon l'invention, dans laquelle $R_4$ est défini comme précédemment, par l'hydrogène en présence d'un catalyseur pour obtenir le composé de formule (If) selon l'invention dans laquelle $R_4$ est défini comme précédemment. Dans chacun des composés

(Ib), (IV) , (Id) , (V) , (Ie) et (If), X est tel que défini dans la formule (I) ci-dessus.

Les composés de formule (I) dans laquelle $R_1$ est un atome d'hydrogène, et $R_2$ est un groupe $OR_3$, où $R_3$ est défini comme dans la formule (I) ci-dessus, peuvent être préparés selon le procédé représenté dans l'annexe 3. Selon ce procédé, on traite le composé de formule (II) par l'un des isomères 4 *(R)* ou 4 *(S)* de la 4-phénylméthoxyméthyl-1,3-dioxolan-2-one (IIIb), en présence de carbonate de potassium, pour obtenir l'isomère 5(*S*) ou *5(R)* du composé de formule (VI). On débenzyle l'isomère de formule (VI) par hydrogénation catalytique pour donner l'isomère 5(S) ou 5(R) du composé de formule (Ib) tel que défini plus haut. On traite ensuite ce dernier par un composé de formule $R_3Y$ dans laquelle $R_3$ est défini comme dans la fomule (I) ci-dessus, à l'exception de la valeur hydrogène, et Y est un groupement partant, tel qu'un atome de chlore ou de brome ou un groupe tosyloxy, pour donner les isomères 5(*S*) ou 5*(R)* des composés de formule (Ic) selon l'invention, dans laquelle $R_3$ est défini comme précédemment. Dans tous les composés (II), (Ib), (Ic), et (VI) définis ci-dessus, X est défini comme dans la formule (I) ci-dessus.

Les composés (Ig) pour lesquels $R_1$ représente un atome d'hydrogène, et $R_2$ est un groupe $-CH=CH-R_4$ ou $-CH_2-CH_2-R_4$, sont préparés par déméthylation des composés (If) selon l'invention, à l'aide du tribromure de bore, selon le procédé décrit en annexe 2.

Le composé de formule (II) est préparé selon le procédé représenté dans l'annexe 4. Selon ce procédé, on traite le 2-fluoro-4-hydroxybenzonitrile, par le bromure de benzyle en présence de carbonate de potassium. On traite ensuite le 2-fluoro-4-phénylméthoxybenzonitrile ainsi obtenu par trois voies différentes selon la signification de X :

- Lorsque X représente un atome d'oxygène : on traite le 2-fluoro-4-phénylméthoxybenzonitrile par l'acétone oxime, en présence de t-butanolate de potassium, pour préparer le 2-[[(1-méthyléthylidène)amino]oxy]-4-phénylméthoxybenzonitrile, que l'on fait réagir avec une solution d'acide chlorhydrique dans l'éthanol,
- Lorsque X représente un atome de soufre : on traite le 2-fluoro-4-phénylméthoxybenzonitrile par du soufre et de l'ammoniac, dans le propanol,
- Lorsque X représente un groupe NR, R étant une chaîne alkyle en $C_1$-$C_4$, linéaire ou ramifiée : on traite le 2-fluoro-4-phénylméthoxybenzonitrile par du $R_5NHNH_2$ où $R_5$ est une chaîne alkyle en $C_1$-$C_4$, linéaire ou ramifiée, dans l'éthanol,

Chacune de ces voies conduit à la préparation d'un composé de formule (VII), où X représente un atome d'oxygène, un atome de soufre ou un groupe NR, R étant une chaîne alkyle en $C_1$-$C_4$, linéaire ou ramifiée. On traite le composé de formule (VII) par le chloroformiate d'éthyle, en présence d'hydrogénocarbonate de sodium, pour donner le composé de formule (II), dans laquelle X est défini comme précédemment.

L'isomère 4*(S)* du composé de formule (IIIa) est un composé connu dont la préparation est décrite dans le brevet EP-0 511 031.

Son isomère 4*(R)* est préparé selon la même méthode, à partir du (*R*)-2,2-diméthyl-1,3-dioxolane-4-méthanol.

L'isomère 4(*S*) du composé 4(*R*)-phénylméthoxyméthyl-1,3-dioxolan-2-one de formule (IIIb) est un composé connu dont la préparation est décrite dans Helvetica Chimica Acta, <u>66</u>, 1210-1240 (1983).

Son isomère 4*(R)* est préparé selon la même méthode, à partir du (*R*)-2,2-dinéthyl-1,3-dioxolane-4-méthanol.

Les exemples suivants illustrent la présente invention.

<u>Exemple 1</u> : <u>(*S*)-5-méthoxyméthyl-3-[1-méthyl-6-(phénylméthoxy)-1*H*-indazol-3-yl]oxazolidin-2-one</u>

1.1. (*R*)-4-méthoxyméthyl-2,2-diméthyl-1,3-dioxolane

Dans un réacteur de 6 litres muni d'un réfrigérant, d'une sonde de température et d'une ampoule d'addition, on introduit 420 ml d'eau déminéralisée et 420 g (10,5 mol) d'hydro-xyde de sodium en pastilles. A la solution agitée à 20°C, on ajoute 2,3 l de dichlorométhane, 396 g (3,00 mol) de *(R)*-2,2-diméthyl-1,3-dioxolane-4-méthanol ($[\alpha]_D^{20}$ = -11° ; c = 4 ; méthanol) et 20,5 g (0,090 mol) de chlorure de benzyltri-éthylammonium. On ajoute ensuite 567 g (4,50 mol) de sulfate de diméthyle en 50 min, en maintenant la température en dessous de 30°C. On agite le mélange pendant 18 heures puis on ajoute 1 litre d'eau. On sépare la phase organique et on la lave avec 0,5 1 d'eau. On réextrait les phases aqueuses avec 3 1 de dichlorométhane puis on réunit les phases orga-niques, on les filtre et on les concentre par distillation sous pression réduite. On obtient 496 g de produit.

1.2. *(S)*-3-méthoxypropane-1,2-diol

On chauffe à 60°C, sous agitation, un mélange des 496 g de produit obtenu à l'étape précédente dans 220 ml d'eau déminéralisée, puis on ajoute 1,5 ml d'acide chlorhydrique à 36 %. On maintient le chauffage pendant 40 mn, puis on amène le milieu à pH 8-9 par addition de 19 ml de triéthylamine. On évapore le solvant sous une pression de 5,2 kPa, à une température inférieure à 70°C puis on distille le résidu à 61°C sous une pression de 13 Pa. On obtient

246 g de produit.
$[\alpha]_D^{20}$ = + 5,8° (c= 4 ; méthanol).

1.3. (*R*)-4-méthoxyméthyl-1,3-dioxolan-2-one

Dans un ballon muni d'une ampoule à addition et d'un montage à distiller, on introduit 245 g (3,31 mol) de (*S*)-3-méthoxy-propane-1,2-diol et 560 ml (4,62 mol) de diéthylcarbonate. On chauffe le mélange à 95°C puis on ajoute une solution de méthylate de sodium obtenue à partir de 10 ml de méthanol et de 0,5 g (0,02 mol) de sodium. On distille pendant 2 heures l'éthanol formé au cours de la réaction (température de masse : 95 à 112°C ; température de colonne : 82 à 78°C), puis on refroidit le mélange et on le distille sous une pression de 13 Pa pour séparer l'excès de diéthylcarbonate. On obtient 267 g de produit.
$[\alpha]_D^{20}$ = + 30,3° (c = 1 ; dichlorométhane).

1.4. 2-Fluoro-4-(phénylméthoxy)benzonitrile

A une solution de 13,3 g (0,106 mol) de 2-fluoro-4-hydroxy-benzonitrile dans 150 ml d'acétonitrile, on ajoute 15,2 ml (0,127 mol) de bromure de benzyle et 29,3 g (0,212 mol) de carbonate de potassium. On agite le mélange au reflux pendant 1 h 30 min, puis on le filtre, on concentre le filtrat sous pression réduite et on dilue l'huile obtenue dans un minimum de dichlorométhane. Après cristallisation par addition d'éther diisopropylique, filtration et séchage, on obtient 20,3 g de produit.
Point de fusion : 87°C.

1.5. 1-méthyl-6-(phénylméthoxy)-1*H*-indazol-3-amine

On fait chauffer à reflux 20 g (0,088 mol) de 2-fluoro-4-(phénylméthoxy)benzonitrile avec 60 ml d'une solution d'éthanol et 15,45 ml (0,29 mol) d'une solution de méthyl-hydrazine pendant 11 heures. On refroidit le mélange à 0°C, puis on collecte le précipité par filtration. Le solide est lavé à l'éthanol puis à l'éther. On obtient 20,2 g de produit.
Point de fusion : 150°C.

1.6. [1-méthyl-6-(phénylméthoxy)-1*H*-indazol-3-yl]carbamate d'éthyle

A une solution de 19,7 g (0,078 mol) de 1-méthyl-6-(phénylméthoxy)-1*H*-indazol-3-amine dans 200 ml d'un mélange de tétrahydrofurane/eau (9/1), on ajoute 9,8 g d'hydrogénocarbonate de sodium (0,117 mol), puis on ajoute goutte à goutte, en maintenant la température à 25°C, 8,9 ml (0,093 mol) de chloroformiate d'éthyle. On obtient une suspension laiteuse, qu'on laisse sous agitation pendant 30 min, puis on évapore le solvant sous pression réduite. On traite le résidu par du dichlorométhane et de l'eau. On décante la phase organique, on la sèche sur du sulfate de sodium, et on la concentre sous pression réduite. Le produit est cristallisé dans l'éther diisopropylique. On obtient 20,1 g de produit.
Point de fusion : 204°C

1.7. (*S*)-5-méthoxyméthyl-3-[1-méthyl-6-(phénylméthoxy)-1*H*-indazol-3-yl]oxazolidin-2-one

On chauffe à 135°C un mélange de 1,03 g (7,8 mmol) de (*R*)-4-méthoxyméthyl-1,3-dioxolan-2-one et 82 mg (0,6 mmol) de carbonate de potassium dans 30 ml de diméthylformamide anhydre, puis on ajoute en 20 mn, une solution de 1,95 g (6 mmol) de [1-méthyl-6-(phénylméthoxy)-1*H*-indazol-3-yl]carbamate d'éthyle dans 30 ml de diméthylformamide. On agite le milieu réactionnel à 135°C pendant 45 min, puis on le refroidit et on évapore le solvant sous pression réduite. On purifie le résidu sur colonne de silice avec un mélange 50/50 d'acétate d'éthyle et de cyclohexane. Le produit est isolé sous forme d'huile qui cristallise et qui est triturée dans l'éther diisopropylique. On obtient 1,5 g de produit.
Point de fusion : 116-117°C
$[\alpha]_D^{20}$ = +26,1 (c=1 ; méthanol)

<u>Exemple 2</u> : <u>(*S*)-3-(6-hydroxy-1-méthyl-1*H*-indazol-3-yl)-5-(méthoxyméthyl)oxazolidin-2-one</u>

On hydrogène 3,1 g (8,4 mmol) de (S)-5-(méthoxyméthyl)-3-[1-méthyl-6-(phénylméthoxy)-1*H*-indazol-3-yl] oxazolidin-2-one dans 40 ml de tétrahydrofurane et 40 ml d'éthanol, en présence de 500 mg de palladium sur charbon à 10% (contenant 50% d'humidité). Après filtration du catalyseur et évaporation du solvant sous pression réduite, on purifie le résidu par chromatographie sur colonne de silice avec du dichlorométhane et on obtient 2,1 g de produit.

Point de fusion : 50-55°C.
$[\alpha]_D^{20}$ = +33,8° (c=1, méthanol)

Exemple 3 : (*S*)-5-méthoxyméthyl-3-[1-méthyl-6-(4,4,4-trifluorobutoxy)-1*H*-indazol-3-yl]oxazolidin-2-one

On agite à reflux pendant 3 h, un mélange de 524 mg (2 mmol) de (*S*)-3-(6-hydroxy-1-méthyl-1*H*-indazol-3-yl)-5-(méthoxyméthyl)oxazolidin-2-one, 478 mg (2,5 mmol) de 4,4,4-trifluoro-1-bromobutane et 552 mg (4 mmol) de carbonate de potassium dans 10 ml d'acétonitrile. On refroidit ensuite le mélange, on le filtre, on évapore le solvant sous pression réduite, et on purifie le résidu par recristallisation dans un mélange isopropanol-éther diisopropylique. On obtient 0,4 g de produit sous forme de poudre blanche.
Point de fusion : 103-104°
$[\alpha]_D^{20}$ = +25,6° (c=1, méthanol)

Exemple 4 : (*S*)-5-méthoxyméthyl-3-[6-(4,4,4-trifluorobutoxy)--1*H*-indazol-3-yl]oxazolidin-2-one

Un mélange de 0,30 g (0,77 mol) de *(S)*-5-méthoxyméthyl-3-[1-méthyl-6-(4,4,4-trifluorobutoxy)-1*H*-indazol-3-yl]oxazolidin-2-one, de 0,47 g (1,9 mol) de peroxyde de benzoyle dans 10 ml de dichlorométhane est porté à reflux pendant 12 h. On évapore le solvant sous pression réduite, on reprend le résidu au méthanol et on filtre l'insoluble. On ajoute ensuite 4 ml de soude à 1N. On agite pendant 15 min. On isole le produit par filtration et on le recristallise dans le *n*-butanol. On obtient 0,20 g du produit.
Point de fusion : 184,9°C-185,3°C
$[\alpha]_D^{20}$ = +11,6° (c = 1 ; diméthylsulfoxyde)

Exemple 5 : (*S*)-5-méthoxyméthyl-3-[6-(phénylméthoxy)-1,2-benzisoxazol-3-yl]oxazolidin-2-one

5.1. 2-[[(1-méthyléthylidène)amino]oxy]-4-(phénylméthoxy) benzonitrile

On agite pendant 30 min, une solution de 7,83 g (0,107 mol) d'acétone oxime dans 200 ml de diméthylformamide, en présence de 12 g (0,11 mol) de t-butanolate de potassium à 95 %. On ajoute ensuite, en 15 min, une solution de 20,3 g (0,089 mol) de 2-fluoro-4-(phénylméthoxy)benzonitrile dans 100 ml de diméthylformamide. On agite le mélange pendant 2 heures puis on le verse dans de l'eau glacée. On filtre le produit cris-tallisé, on le dissout dans du dichlorométhane, puis on sèche la solution sur sulfate de sodium et on la concentre sous pression réduite. On obtient 21,2 g de produit.
Point de fusion : 102°C.

5.2. 6-(phénylméthoxy)-1,2-benzisoxazol-3-ylamine

On fait réagir 20,2 g (0,072 mol) de 2-[[(1-méthyléthyl idène)amino]oxy]-4-(phénylméthoxy)benzonitrile avec 340 ml d'une solution 4N d'acide chlorhydrique dans l'éthanol pendant 20 heures, puis on évapore le solvant. On triture ensuite le produit cristallisé dans du dichlorométhane, puis on filtre et on dissout le solide dans un minimum de méthanol tiède. On alcalinise la solution avec de l'ammoniaque, puis on dilue avec de l'eau. Après filtration et lavage à l'eau, on obtient 16,3 g de produit.
Point de fusion : 166°C.

5.3. [6-(phénylméthoxy)-1,2-benzisoxazol-3-yl]carbamate d'éthyle

A une solution de 10,1 g (0,042 mol) de 6-(phénylméthoxy)-1,2-benzisoxazol-3-amine dans 100 ml d'un mélange 9/1 de tétrahydrofurane et d'eau, on ajoute 8,8 ml (0,092 mol) de chloroformiate d'éthyle et 10,6 g (0,126 mol) d'hydrogénocarbonate de sodium. On agite le mélange pendant 18 heures, puis on évapore le solvant et on traite le résidu par du dichlorométhane et de l'eau. On décante la phase organique, on la sèche sur sulfate de sodium et on la concentre sous pression réduite. Après cristallisation dans l'alcool isopropylique et recristallisation dans le n-butanol, on obtient 11,5 g de produit.
Point de fusion : 144-146°C.

5.4. *(S)*-5-méthoxyméthyl-3-[6-(phénylméthoxy)-1,2-benzisoxazol-3-yl]oxazolidin-2-one

On porte à 140°C, un mélange de 4,5 g (0,034 mol) de 4(*R*)-méthoxyméthyl-1,3-dioxolan-2-one et 0,24 g (1,7 mmol) de carbonate de potassium dans 35 ml de diméthylformamide anhydre puis on ajoute, en 20 mn, une solution

de 5,5 g (18 mmol) de 6-(phénylméthoxy)-1,2-benzisoxazol-3-carbamate d'éthyle dans 20 ml de diméthylformamide. On agite le milieu à 140°C pendant 40 mn, puis on le refroidit et on évapore le solvant sous pression réduite. On purifie le résidu sur colonne de silice avec un mélange 30/70 d'acétate d'éthyle et de cyclohexane. Après cristallisation dans l'éther diisopropylique, on obtient 4,1 g de produit. Point de fusion : 92,0-92,1°C.
$[\alpha]_D^{20}$ = + 8,6° (c = 1 ; dichlorométhane).

Exemple 6 : (*S*)-3-(6-hydroxy-1,2-benzisoxazol-3-yl)-5-(méthoxyméthyl)oxazolidin-2-one

On hydrogène pendant 30 mn, une solution de 3,9 g (0,011 mol) de (*S*)-5-méthoxyméthyl-3-[6-(phénylméthoxy)-1,2-benzisoxazol-3-yl]oxazolidin-2-one dans 60 ml de tétrahydrofurane et 60 ml d'éthanol, en présence de 1,1 g de palladium sur charbon à 5 % (contenant 50 % d'humidité). On filtre ensuite le cata-lyseur et on évapore le filtrat sous pression réduite. On obtient 2,3 g de produit. Point de fusion : 148,7-148,8°C.
$[\alpha]_D^{20}$ = + 14,2 ° (c = 1 ; diméthylsulfoxyde).

Exemple 7 : (*S*)-5-méthoxyméthyl-3-[6-(4,4,4-trifluorobutoxy)-1,2-benzisoxazol-3-yl]oxazolidin-2-one

On agite à reflux pendant 30 mn, un mélange de 1,3 g (4,9 mmol) de *(S)*-3-(6-hydroxy-1,2-benzisoxazol-3-yl)-5-méthoxyméthyl-oxazolidin-2-one, 1,4 g (7,3 mmol) de 4-bromo-1,1,1-trifluorobutane et 1,4 g (9,8 mmol) de carbonate de potassium dans 20 ml d'acétonitrile. On refroidit ensuite le mélange, on le filtre, on évapore le solvant sous pression réduite et on purifie le résidu par chromatographie sur colonne de silice avec du dichlorométhane. Après traitement au noir végétal dans du dichlorométhane, on obtient 1,5 g de produit.
Point de fusion : 120,4-120,5°C.
$[\alpha]_D^{20}$ = + 8,7° (c = 1 ; dichlorométhane).

Exemple 8 : (*R*)-5-méthoxyméthyl-3-[6-(phénylméthoxy)-1,2-benzisoxazol-3-yl]oxazolidin-2-one

On porte à 140°C, un mélange de 1,4 g (0,010 mol) de 4(S)-méthoxyméthyl-1,3-dioxolan-2-one et 0,1 g (0,00073 mol) de carbonate de potassium dans 35 ml de diméthylformamide anhydre puis on ajoute, en 20 min, une solution de 2,5 g (0,0080 mol) de 6-(phénylméthoxy)-1,2-benzisoxazol-3-carbamate d'éthyle dans 10 ml de diméthylformamide. On agite le milieu à 140°C pendant 35 min, puis on le refroidit et on évapore le solvant sous pression réduite. On purifie le résidu sur colonne de silice avec un mélange 25/75 d'acétate d'éthyle et de cyclohexane. Après cristallisation dans l'éther diisopropylique, on obtient 1,65 g de produit.
Point de fusion : 92,0-92,2°C.
$[\alpha]_D^{20}$ = - 9,6° (c = 1 ; dichlorométhane).

Exemple 9 : (*R*)-3-(6-hydroxy-1,2-benzisoxazol-3-yl)-5-(méthoxyméthyl)oxazolidin-2-one

On hydrogène pendant 30 min, une solution de 21 g (0,059 mol) de *(R)*-5-méthoxyméthyl-3-[6-(phénylméthoxy)-1,2-benzisoxazol-3-yl]oxazolidin-2-one dans 310 ml de tétrahydrofurane et 310 ml d'éthanol, en présence de 6 g de palladium sur charbon à 5 % (contenant 50 % d'humidité). On filtre ensuite le cata-lyseur et on évapore le filtrat sous pression réduite. On purifie le résidu sur colonne de silice avec un mélange à 2 % de méthanol dans du dichlorométhane. On obtient 2,3 g de produit.
Point de fusion : 151,4-151,5°C.
$[\alpha]_D^{20}$ = - 14,2 ° (c = 1 ; diméthylsulfoxyde).

Exemple 10 : (*R*)-5-méthoxyméthyl-3-[6-(4,4,4-trifluoro-3(*R*)-hydroxybutoxy)-1,2-benzisoxazol-3-yl]oxazolidin-2-one

On agite au reflux pendant 3 heures, un mélange de 1,0 g (3,8 mmol) de *(R)*-3-(6-hydroxy-1,2-benzisoxazol-3-yl)-5-(méthoxyméthyl)-oxazolidin-2-one, 1,8 g (6,2 mmol) de tosylate de 4,4,4-trifluoro-3(*R*)-hydroxybutyle et 1,0 g (7,6 mmol) de carbonate de potassium dans 25 ml d'acétonitrile. On refroidit ensuite le mélange, on évapore le solvant sous pression réduite, on reprend le résidu avec de l'acétate d'éthyle puis on le lave à l'eau. On sèche la phase organique sur sulfate de sodium, on la concentre sous pression réduite et on chromatographie le produit obtenu sur colonne de silice avec un mélange à 1 % de méthanol dans du dichlorométhane. Après recristallisation dans un mélange d'acétate d'éthyle et d'éther diisopropylique, on obtient 0,6 g de produit.
Point de fusion : 147°C.
$[\alpha]_D^{20}$ = + 16,6° (c = 1 ; dichlorométhane).

Exemple 11 : (*S*)-5-hydroxyméthyl-3-[6-(4,4,4-trifluorobutoxy)-1,2-benzisoxazol-3-yl]oxazolidin-2-one

11.1. (*S*)-5-phénylméthoxyméthyl-3-[6-(phénylméthoxy)-1,2-benzisoxazol-3-yl]oxazolidin-2-one

On chauffe à 140°C un mélange de 4,2 g (0,02 mol) de *(R)*-4-phénylméthoxyméthyl-1,3-dioxolan-2-one et 0,14 g (1,0 mmol) de carbonate de potassium dans 50 ml de diméthylformamide anhydre, puis on ajoute en 10 min, une solution de 3,1 g (1,0 mmol) de 6-(phénylméthoxy)-1,2-benzisoxazol-3-carbamate d'éthyle dans 10 ml de diméthylformamide. On agite le milieu réactionnel à 140°C pendant 30 mn, puis on le refroidit et on évapore le solvant sous pression réduite. On purifie le résidu sur colonne de silice avec un mélange à 20 % d'acétate d'éthyle dans du cyclohexane. On obtient 2,0 g de produit.
Point de fusion : 98-99°C.
$[\alpha]_D^{20}$ = - 1,0° (c = 1 ; dichlorométhane).

11.2. (*S*)-5-hydroxyméthyl-3-(6-hydroxy-1,2-benzisoxazol-3-yl)oxazolidin-2-one

On hydrogène pendant 50 min, une solution de 1,7 g (0,040 mol) de (*S*)-5-phénylméthoxyméthyl-3-[6-(phénylméthoxy)-1,2-benzisoxazol-3-yl]oxazolidin-2-one dans 20 ml de tétrahydro-furane et 2 ml d'une solution 3N d'acide chlorhydrique dans l'éthanol, en présence de 0,5 g de palladium sur charbon à 5 % (contenant 50 % d'humidité). On filtre ensuite le cata-lyseur et on évapore le filtrat sous pression réduite. Après trituration du résidu dans du dichlorométhane, on obtient 0,85 g de produit.
Point de fusion : 226-228°C.
$[\alpha]_D^{20}$ = + 18,1° (c = 1 ; diméthylsulfoxyde).

11.3. (*S*)-5-hydroxyméthyl-3-[6-(4,4,4-trifluorobutoxy)-1,2-benzisoxazol-3-yl]oxazolidin-2-one

On agite à reflux pendant 1 heure, un mélange de 0,75 g (0,030 mol) de *(S)*-5-hydroxyméthyl-3-(6-hydroxy-1,2-benziso-xazol-3-yl)oxazolidin-2-one, 0,63 g (3,3 mmol) de 4-bromo-1,1,1-trifluorobutane et 0,83 g (6,0 mmol) de carbonate de potassium dans 12 ml de diméthylformamide et 2,5 ml d'acéto-nitrile, puis on le refroidit, on le verse dans de l'eau et on filtre le précipité. Après lavage à l'eau puis à l'éther de pétrole et chromatographie sur colonne de silice avec un mélange à 50 % d'acétate d'éthyle dans du dichlorométhane, on obtient 0,70 g de produit. Point de fusion : 159,6-159,9°C.
$[\alpha]_D^{20}$ = + 12,1° (c= 1; dichlorométhane).

Exemple 12 : (*R*)-5-(méthoxyméthyl)-3-(6-éthènyl-1,2-benzisoxazol-3-yl)oxazolidin-2-one

12.1. trifluorométhanesulfonate de [(*R*)-5-(méthoxyméthyl)-2-oxo-3-oxazolidinyl]-1,2-benzisoxazol-6-yle

A une solution de 11 g (0,042 mol) de (*R*)-5-(méthoxyméthyl)-3-(6-hydroxy-1,2-benzisoxazol-3-yl)oxazolidin-2-one dans 110 ml de pyridine, on ajoute à 0°C, en 10 min, 8,4 ml (0,050 mol) d'anhydride trifluorométhanesulfonique. On agite la solution pendant 18 heures puis on la verse dans une solution aqueuse d'acide chlorhydrique 2N glacée. On extrait le produit avec de l'acétate d'éthyle puis on sèche la phase organique sur sulfate de sodium et on la concentre sous pression réduite. On obtient 16,5 g de produit.
Point de fusion : 94°C.

12.2. *(R)*-5-(méthoxyméthyl)-3-(6-éthènyl-1,2-benzisoxazol-3-yl)oxazolidin-2-one

On agite pendant 2 heures, à reflux, un mélange de 14,9 g (0,038 mol) de trifluorométhanesulfonate de [(*R*)-5-(méthoxy-méthyl)-2-oxo-3-oxazolidinyl]-1,2-benzisoxazol-6-yle, 12,3 g (0,038 mol) de tributylvinylétain, 765 mg (0,66 mmol) de tétrakis(triphénylphoshine)palladium et 4,8 g (0,11 mol) de chlorure de lithium dans 160 ml de dioxane. On évapore ensuite le solvant sous pression réduite, on reprend le résidu avec de l'acétate d'éthyle, on filtre sur silice, on lave la phase organique avec de l'eau, on la sèche sur sul-fate de sodium et on la concentre sous pression réduite. On redissout l'huile obtenue dans de l'acétonitrile, on lave avec de l'hexane et on concentre la solution. Par chromatographie du résidu sur colonne de silice avec un mélange à 30 % d'acétate d'éthyle dans du cyclohexane, on obtient 16,5 g de produit.
Point de fusion : 79,2-79,4°C.
$[\alpha]_D^{20}$ = - 5,4° (c = 1 ; dichlorométhane)

Exemple 13 : trans-(*R*)-5-(méthoxyméthyl)-3-[6-(5,5,5-trifluoro-4(*R*)-hydroxypent-1-ényl)-1,2-benzisoxazol-3-yl]oxazolidin-2-one

13.1. (*R*)-5-Méthoxyméthyl-3-(6-formyl-1,2-benzisoxazol-3-yl)oxazolidin-2-one

On fait passer un courant d'ozone à - 40°C, pendant 2 heures, dans une solution de 8,0 g (0,029 mol) de (*R*)-5-(méthoxy-méthyl)-3-(6-éthènyl-1,2-benzisoxazol-3-yl)oxazolidin-2-one dans 210 ml de dichlorométhane, puis on chasse l'ozone par un courant d'argon et on ajoute 10,7 ml (0,15 mol) de sulfure de diméthyle. On agite pendant 3 heures en laissant la température remonter jusqu'à la température ambiante, puis on évapore le solvant sous pression réduite. Par chromatographie du résidu sur colonne de silice avec un mélange à 30 % d'acétate d'éthyle dans du cyclohexane, on obtient 5,0 g de produit.
Point de fusion : 116°C.

13.2. trans-(*R*)-5-(méthoxyméthyl)-3-[6-(5,5,5-trifluoro-4(*R*)-hydroxypent-1-ényl)-1,2-benzisoxazol-3-yl]oxazolidin-2-one

On agite à reflux pendant 2 heures, un mélange de 1,8 g (6,5 mmol) de *(R)*-5-méthoxyméthyl-3-(6-formyl-1,2-benzisoxazol-3-yl]oxazolidin-2-one, 4,0 g (7,8 mmol) d'iodure de (4,4,4-trifluoro-3(R)-hydroxybutyl)triphényl-phosphonium et 1,25 g (9,1 mmol) de carbonate de potassium dans 1,4 ml de formamide et 18 ml de dioxane puis on le verse dans de l'eau glacée. On extrait ensuite le produit avec de l'acétate d'éthyle, on sèche la phase organique sur sulfate de sodium et on la concentre sous pression réduite. Après chromatographie du résidu sur colonne de silice avec un mélange à 30 % d'acétate d'éthyle dans du cyclohexane et trituration dans l'éther diisopropylique, on obtient 1,2 g de produit.
Point de fusion : 141,1-141,6°C.
$[\alpha]_D^{20}$ = + 15,8° (c = 1 ; dichlorométhane)

Exemple 14 : *(R)*-5-(méthoxyméthyl)-3-[6-(5,5,5-trifluoro-4(*R*)-hydroxypentyl)-1,2-benzisoxazol-3-yl]oxazolidin-2-one

On hydrogène pendant 30 min un mélange de 1,0 g (0,0026 mol) de trans-*(R)*-5-(méthoxyméthyl)-3-[6-(5,5,5-trifluoro-4*(R)*-hydroxy-1-pentènyl)-1,2-benzisoxazol-3-yl]oxazolidin-2-one dans 30 ml d'éthanol, en présence de 0,22 g de palladium sur charbon à 5 % contenant 50 % d'eau. Après filtration, on concentre le filtrat sous pression réduite. Par chromatogra-phie du résidu sur colonne de silice avec un mélange à 1,5 % de méthanol dans du dichlorométhane et trituration dans un mélange d'éther de pétrole et d'éther diisopropylique, on obtient 0,88 g de produit.
Point de fusion : 129,0-129,4°C.
$[\alpha]_D^{20}$ = + 4,9° (c = 1 ; dichlorométhane).

Exemple 15 : (*R*)-5-hydroxyméthyl-3-[6-(5,5,5-4(*R*)hydroxypentyl)-1,2-benzisoxazol-3-yl]oxazolidin-2-one

A une solution de 0,495 g (1,27 mmol) de (*R*)-5-(méthoxyméthyl)-3-[6-(5,5,5-trifluoro-4(*R*)-hydroxypentyl)-1,2-benzisoxazol-3-yl]oxazolidin-2-one dans 5 ml de dichlorométhane sont ajoutés goutte à goutte, à 0°C, 3,8 ml (3,8 mmol) d'une solution 1M de tribromure de bore dans le dichlorométhane. Après 2 h de réaction, le milieu est dilué avec du dichlorométhane et traité avec de l'ammoniaque dilué jusqu'à un pH légèrement basique. La phase organique est séparée, séchée sur sulfate de sodium et évaporée. Par chromatographie sur colonne de silice avec un mélange d'acétate d'éthyle et de cyclohexane puis trituration dans l'acétate d'éthyle, on obtient 0,12 g de produit.
Point de fusion : 135,1-136,2 °C
$[\alpha]_D^{20}$ = 0,0° (c = 1 ; méthanol).

Exemple 16 : (*S*)-5-méthoxyméthyl-3-[6-(phénylméthoxy)-1,2-benzisothiazol-3-yl]oxazolidin-2-one

16.1. 6-(phénylméthoxy)-1,2-benzisothiazol-3-amine

On porte pendant 5h, dans un autoclave à 100°C, un mélange de 13,2 g (0,058 mol) de 2-fluoro-4-(phénylméthoxy)benzonitrile et de 1,85 g (0,058 mol) de soufre, dans 15 ml (0,58 mol) d'ammoniac et 50 ml de méthylglycol. On évapore ensuite le méthylglycol sous pression réduite. On reprend le mélange par du dichlorométhane, on filtre l'insoluble, puis on évapore le solvant sous pression réduite. Le produit est purifié par chromatographie sur colonne de silice avec du cyclohexane et de l'acétate d'éthyle, dans des proportions 60/40. Puis une deuxième purification par chromatographie sur colonne de silice avec un mélange d'éther diisopropylique et de méthanol dans des proportions 99/1 conduit à 1,7 g de produit.

Point de fusion : 158°C

16.2. [6-(phénylméthoxy)-1,2-benzisothiazol-3-yl]carbamate d'éthyle

Selon le procédé de l'exemple 1.6., on obtient 1,19 g de [6-(phénylméthoxy)-1,2-benzisothiazol-3-yl]carbamate d'éthyle, à partir de 1,28 g (0,005 mol) de 6-(phénylméthoxy)-1,2-benzisothiazol-3-amine.
Point de fusion : 149-150°C

16.3. (*S*)-5-méthoxyméthyl-3-[6-(phénylméthoxy)-1,2-benzisothiazol-3-yl]oxazolidin-2-one

Selon le procédé décrit à l'exemple 1.7., on obtient 0,4 g de (S)-5-méthoxyméthyl-3-[6-(phénylméthoxy)-1,2-benzisothiazol-3-yl]oxazolidin-2-one, à partir de 0,57 g (1,73 mmol) de [6-(phénylméthoxy)-1,2-benzisothiazol-3-yl] carbamate d'éthyle, 0,29 g (2,2 mmol) de (*R*)-4-méthoxyméthyl-1,3-dioxolan-2-one, et de 24 mg (0,17 mmol) de carbonate de potassium.
Point de fusion : 105-106°C
$[\alpha]_D^{20}$ = + 9,9° (c = 1 ; méthanol)

<u>Exemple 17</u> : <u>(*S*)-5-méthoxyméthyl-3-[6-(4,4,4-trifluorobutoxy)-1,2-benzisothiazol-3-yl]oxazolidin-2-one</u>

17.1. (*S*)-5-méthoxyméthyl-3-(6-hydroxy-1,2-benzisothiazol-3-yl)oxazolidin-2-one

A la solution de 2,10 g (5,67 mmol) de (*S*)-5-méthoxyméthyl-3-[6-(phénylméthoxy)-1,2-benzisothiazol-3-yl] oxazolidin-2-one dans 76 ml de dichlorométhane, refroidie à -8°C, on ajoute, en 4 h, 8,7 ml (68 mmol) de diméthylaniline et 6,9 g (0,051 mol) de chlorure d'aluminium en trois fois. Le mélange est versé sur de l'eau glacée et le produit est extrait avec du dichlorométhane. La phase organique est séchée sur du sulfate de sodium, et concentrée sous pression réduite. Après purification par chromatographie sur colonne de silice avec du dichlorométhane et du méthanol, dans des proportions 99/1, et trituration dans l'éther diisopropylique, on obtient 1,4 g de produit.
Point de fusion : 142-142°C

17.2. (*S*)-5-méthoxyméthyl-3-[6-(4,4,4-trifluorobutoxy)-1,2-benzisothiazol-3-yl]oxazolidin-2-one

Selon le procédé de l'exemple 3., on obtient 0,42 g de (*S*)-5-méthoxyméthyl-3-[6-(4,4,4-trifluorobutoxy)-1,2-benzisothiazol-3-yl]oxazolidin-2-one, à partir de 0,4 g (1,43 mmol) de *(S)*-5-méthoxyméthyl-3-(6-hydroxy-1,2-benzisothiazol-3-yl)oxazolidin-2-one, 0,34 g (1,25 mmol) de bromure de 4,4,4-trifluorobutyle et de 0,42 g (3,1 mmol) de carbonate de potassium dans 8 ml d'acétonitrile.
Point de fusion : 78-79°C
$[\alpha]_D^{20}$ = + 8,9° (c = 1 ; méthanol).
Le tableau suivant rassemble quelques composés selon l'invention, avec leurs caractéristiques physiques.
La configuration notée *R* et/ou *S* ainsi que *5R* et/ou *5S* se réfère à l'hétérocycle oxazolidinone, et la configuration notée *3R, 3S*, et *4R* se réfère à la chaîne $R_2$.

## Tableau

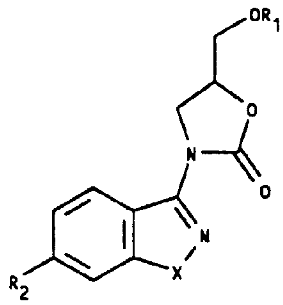

(I)

| N° | X | $R_1$ | $R_2$ | Config. | F (°C) | $[\alpha]_D^{20}$ c=1 ; $CH_2Cl_2$ |
|---|---|---|---|---|---|---|
| 1 | O | Me | Bn-O | *R* | 92,0-92,2 | -9,6° |
| 2 | O | Me | Bn-O | *S* | 92,0-92,1 | +8,6° |
| 3 | O | Me | H-O | *R* | 151,4-151,5 | -14,2°* |
| 4 | O | Me | H-O | *S* | 148,7-148,8 | +14,2°* |
| 5 | O | Me | $F_3C-CHOH-(CH_2)_2-O$ | 3*R*,5*R* | 147,0 | +16,6 |
| 6 | O | Me | 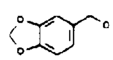 | *R* | 106,0-107,2 | -7,5° |
| 7 | O | H | H-O | *S* | 226-228 | +18,1°* |
| 8 | O | H | Bn-O | *R* | 166,3-166,8 | -14,0° |
| 9 | O | H | $F_3C-(CH_2)_3-O$ | *S* | 159,6-159,9 | +12,1° |
| 10 | O | Me | $F_3C-(CH_2)_3-O$ | *S* | 120,4-120,5 | +8,7° |
| 11 | O | Me | $F_3C-(CH_2)_3-O$ | *R* | 132-133 | -11,2° |
| 12 | O | Me | 3-Cl-Bn-O | *R* | 143,5-144,1 | -7,5° |
| 13 | O | Me | 4-Cl-Bn-O | *R* | 178,7 | -8,6° |
| 14 | O | Me | 4-F-Bn-O | *R* | 145,8-146,0 | -8,6° |
| 15 | O | Me | $4-NO_2-Bn-O$ | *R* | 189,9-190,0 | -9,1° |
| 16 | O | Me | $F_3C-CH=CH-CH_2-O$ | *R*,trans | 120,8-121,1 | -8,7° |
| 17 | O | Me | $CH_3-(CH_2)_3-O$ | *R* | 92,1-92,2 | -10,0° |
| 18 | O | Me | $CH_3-O-(CH_2)_2-O$ | *R* | 94-95 | -9,7° |
| 19 | O | Me | $CH_2=CH$ | *R* | 79,2-79,4 | -5,4° |
| 20 | O | Me | $F_3C-CHOH-CH_2-$ CH=CH | *4R,5R, trans* | 141,1-141,6 | +15,8° |
| 21 | O | Me | $F_3C-CHOH-CH_2-$ CH=CH | *4R,5S, trans* | 130,1-130,3 | +21,2° |
| 22 | O | Me | $F_3C-CHOH-(CH_2)_3$ | *4R,5R* | 129,0-129,4 | +4,9° |
| 23 | O | Me | $F_3C-CHOH-(CH_2)_3$ | *4R,5S* | 111,3-111,7 | +19,8° |
| 24 | O | H | $F_3C-CHOH-(CH_2)_3$ | *4R,5R* | 135,1-136,2 | 0,0°** |
| 25 | O | Me | Ph-CH=CH | *R,trans* | 167,2 | +2,7 |
| 26 | O | Me | Ph-CH=CH | *R,cis* | 53-58 | -3,1° |

| N° | X | $R_1$ | $R_2$ | Config. | F (°C) | $[\alpha]_D^{20}$ c=1 ; $CH_2Cl_2$ |
|---|---|---|---|---|---|---|
| 27 | O | Me | $Ph-CH_2-CH_2$ | *R* | 88,0-88,2 | -8,9° |
| 28 | O | Me | $F_3C-(CH_2)_2-CH=CH$ | *R,cis* | 67,0 | -7,4° |
| 29 | O | Me | $F_3C-(CH_2)_2-CH=CH$ | *S,cis* | 67,1-67,8 | +6,9° |
| 30 | O | Me | $F_3C-(CH_2)_4$ | *R* | 76,7-76,8 | -7,9° |
| 31 | O | Me | $F_3C-(CH_2)_4$ | *S* | 71,6-72,1 | +7,8° |
| 32 | S | Me | Bn-O | *R* | 104-105 | -10,2°** |
| 33 | S | Me | Bn-O | *S* | 105-106 | +9,9°** |
| 34 | S | Me | $F_3C-CHOH-(CH_2)_2-O$ | *3R,5R* | 80-82 | +14,9°** |
| 35 | S | Me | $F_3C-CHOH-(CH_2)_2-O$ | *3R,5S* | 98-99 | +35,2°** |
| 36 | S | Me | $F_3C-(CH_2)_3-O$ | *R* | 79-80 | -9,7°** |
| 37 | S | Me | $F_3C-(CH_2)_3-O$ | *S* | 78-79 | +8,9°** |
| 38 | NMe | Me | H-O | *R* | 50-55 | -32,8°** |
| 39 | NMe | Me | H-O | *S* | 50-55 | +33,8°** |
| 40 | NMe | Me | Bn-O | *R* | 116-117 | -26,1°** |
| 41 | NMe | Me | Bn-O | *S* | 116-117 | +26,1°** |
| 42 | NMe | Me | $F_3C-(CH_2)_3-O$ | *R* | 104-105 | -21,8°** |
| 43 | NMe | Me | $F_3C-(CH_2)_3-O$ | *S* | 103-104 | +25,6°** |
| 44 | NMe | Me | $F_3C-CHOH-(CH_2)_2-O$ | *3R,5R* | 135-136 | 0,0°** |
| 45 | NMe | Me | $F_3C-CHOH-(CH_2)_2-O$ | *3R,5S* | 98-100 | +48,0°** |
| 46 | NH | Me | $F_3C-(CH_2)_3-O$ | *R* | 184,7-185,0 | -10,5°* |
| 47 | NH | Me | $F_3C-(CH_2)_3-O$ | *S* | 184,9-185,3 | +11,6°* |

* : c=1 ; Diméthylsulfoxyde

** : c=1 ; Méthanol

Les composés de l'invention ont fait l'objet d'essais pharmacologiques permettant de déterminer leur pouvoir inhibiteur de la monoamine oxydase A et de la monoamine oxydase B.

Les mesures des activités MAO-A et MAO-B in vitro ont été réalisées en utilisant comme source d'enzyme un homogénat de cerveau de rat, selon la méthode décrite par C. Fowler et M. Strolin-Benedetti, dans J. Neurochem., 40, 1534-1541 (1983).

Le dosage standard consiste à homogénéiser le cerveau de rat dans 20 volumes de tampon phosphate 0,1 M (pH = 7,4) et à préincuber 100 µl d'homogénat (5 mg de tissu) à 37°C pendant 20 minutes, en l'absence ou en présence de différentes concentrations en inhibiteur étudié. La réaction est démarrée par l'addition de $[^{14}C]$sérotonine ($[^{14}C]$5HT, concentration finale 125 µM) pour la mesure de l'activité de la MAO-A ou de $[^{14}C]$phényléthylamine ($[^{14}C]$PEA, concentration finale 8 µM) pour la mesure de l'activité MAO-B, dans un volume final de 500 µl. Après 5 minutes d'incubation pour la $[^{14}C]$5HT et
1 minute d'incubation pour la $[^{14}C]$PEA, la réaction est arrêtée par addition de 200 µl d'acide chlorhydrique 4N. Les métabolites radioactifs issus de la désamination oxydative sont alors séparés du substrat non transformé, par extraction en phase organique, et quantifiés par comptage de la radio-activité.
Les activités inhibitrices vis à vis de la MAO-A et de la MAO-B sont données respectivement par les constantes d'inhibition Ki (MAO-A) et Ki (MAO-B).
Pour les composés de l'invention, les Ki (MAO-A) varient entre 15 nM et des valeurs supérieures à 1 µM et les Ki (MAO-B) entre 1 nM et des valeurs supérieures à 1 µM.

Certains composés de l'invention sont des inhibiteurs sélectifs de la MAO-B, le rapport Ki(MAO-A)/Ki(MAO-B) pouvant être de l'ordre de $10^3$.

D'autres sont cependant des inhibiteurs mixtes de la MAO-A et de la MAO-B, le rapport Ki(MAO-A)/Ki(MAO-B) pouvant être inférieur à 10.

Les résultats obtenus montrent que les composés de l'invention peuvent être utilisés pour la préparation de médicaments inhibiteurs sélectifs de la MAO-B ou inhibiteurs mixtes de la MAO-A et de la MAO-B, ces médicaments trouvant leur emploi en thérapeutique notamment dans le traitement des états dépressifs de toute nature, les psychoses dépressives séniles, l'hypobulia, les phobies sociales, les troubles de l'humeur, dans l'amélioration des performances cérébrales générales, dans la prévention et le traitement des maladies neurodégénératives comme la maladie de Parkinson, la maladie d'Alzheimer et tous les troubles de la mémoire, dans l'anxiété, les attaques de panique, le traitement de la dépendance et du sevrage liés à la consommation de tabac, d'alcool, et/ou de stupéfiants, et la perte d'appétit.

Les composés de l'invention peuvent être présentés, en association avec des excipients, sous forme de compositions formulées en vue de l'administration par voie orale, parentérale ou rectale, par exemple sous forme de comprimés, dragées, capsules, solutions, suspensions ou suppositoires.

Par voie orale, la dose de principe actif administrée peut atteindre 50 mg/kg/jour, en une ou plusieurs prises. Par voies parentérale et rectale, elle peut atteindre 10 mg/kg/jour, en une ou plusieurs prises.

## Annexe 1

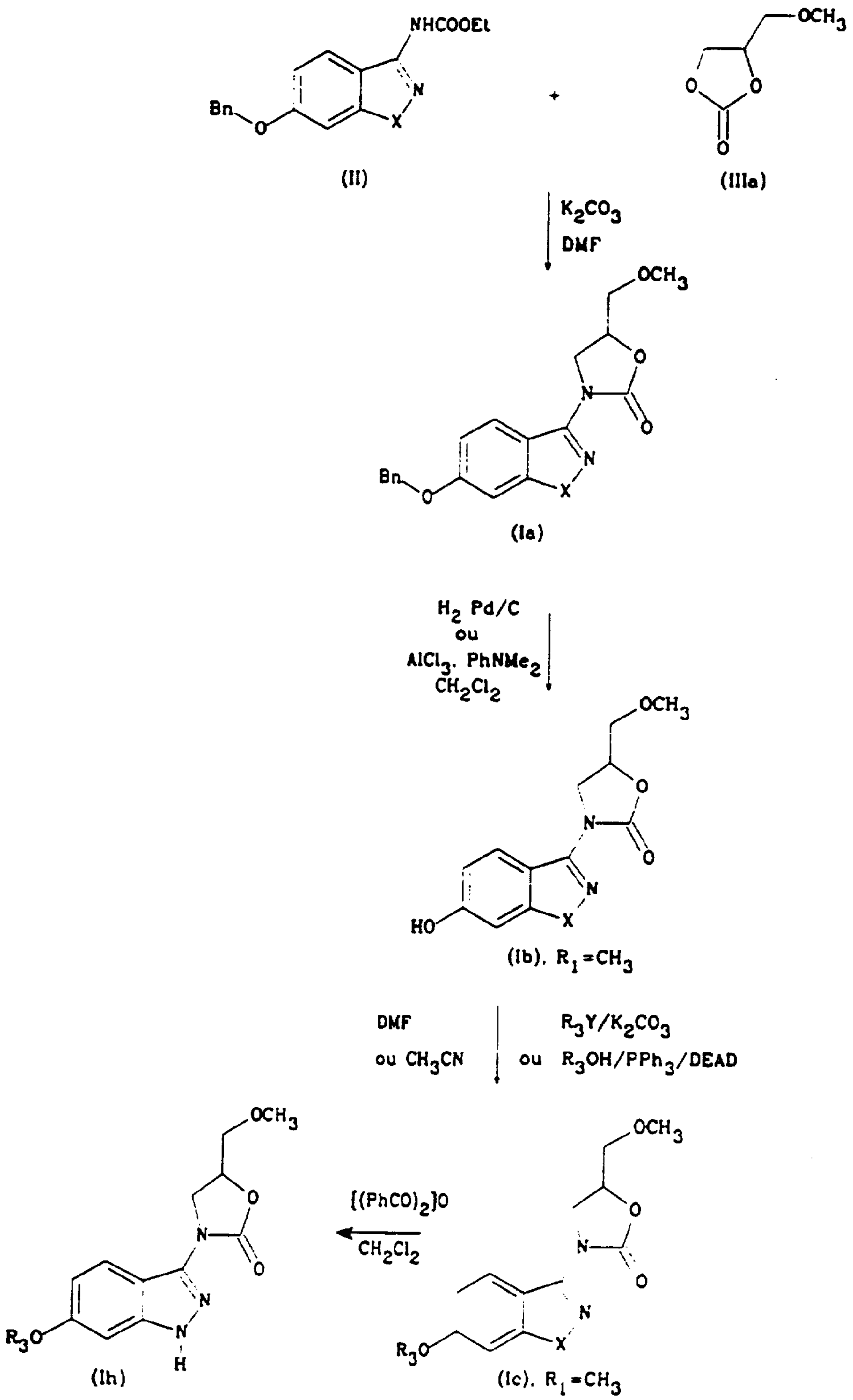


(II)

(IIIa)

(Ia)

(Ib), $R_1 = CH_3$

(Ih)

(Ic), $R_1 = CH_3$

## Annexe 2

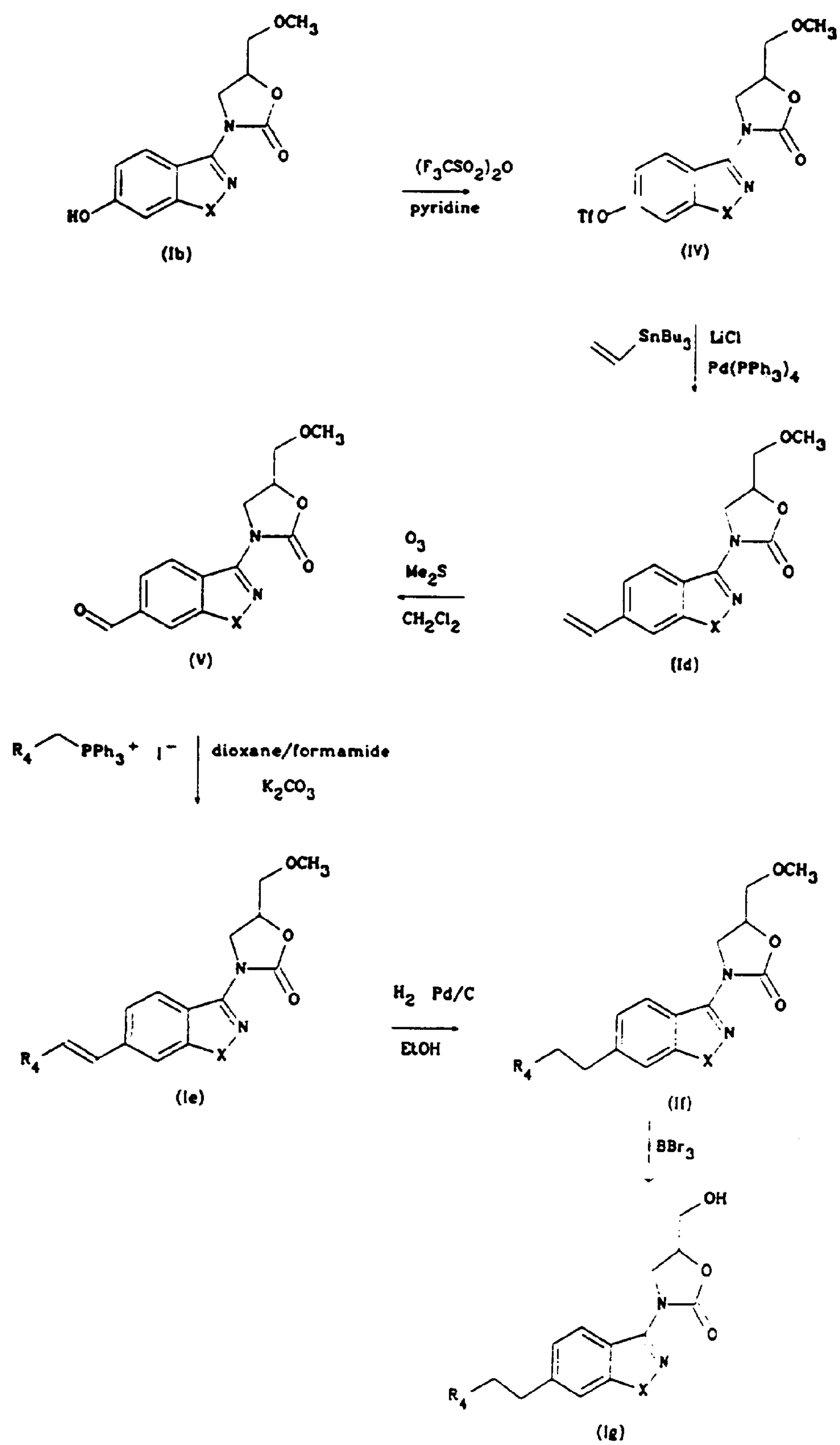


(Ib) (IV) (V) (Id) (Ie) (If) (Ig)

## Annexe 3

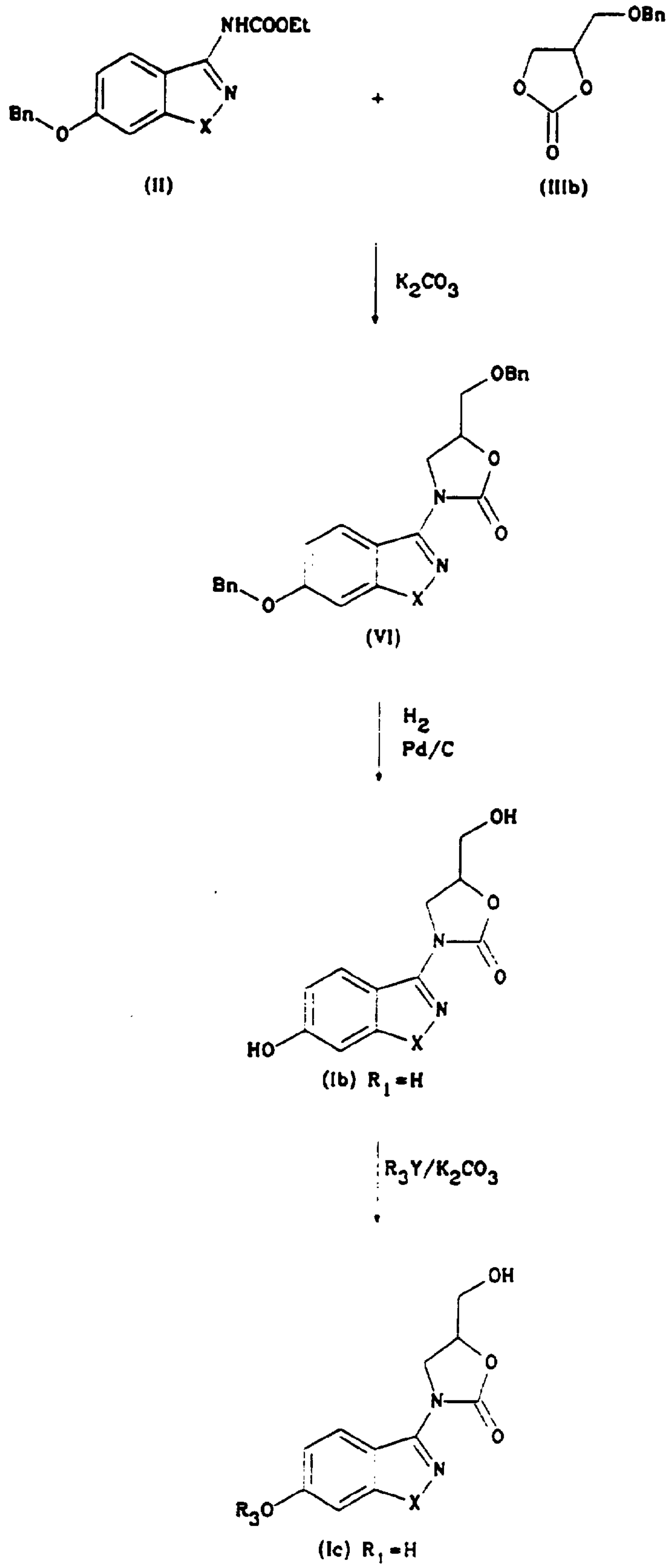


(II) + (IIIb)

(VI)

(Ib) $R_1$ = H

(Ic) $R_1$ = H

## Annexe 4

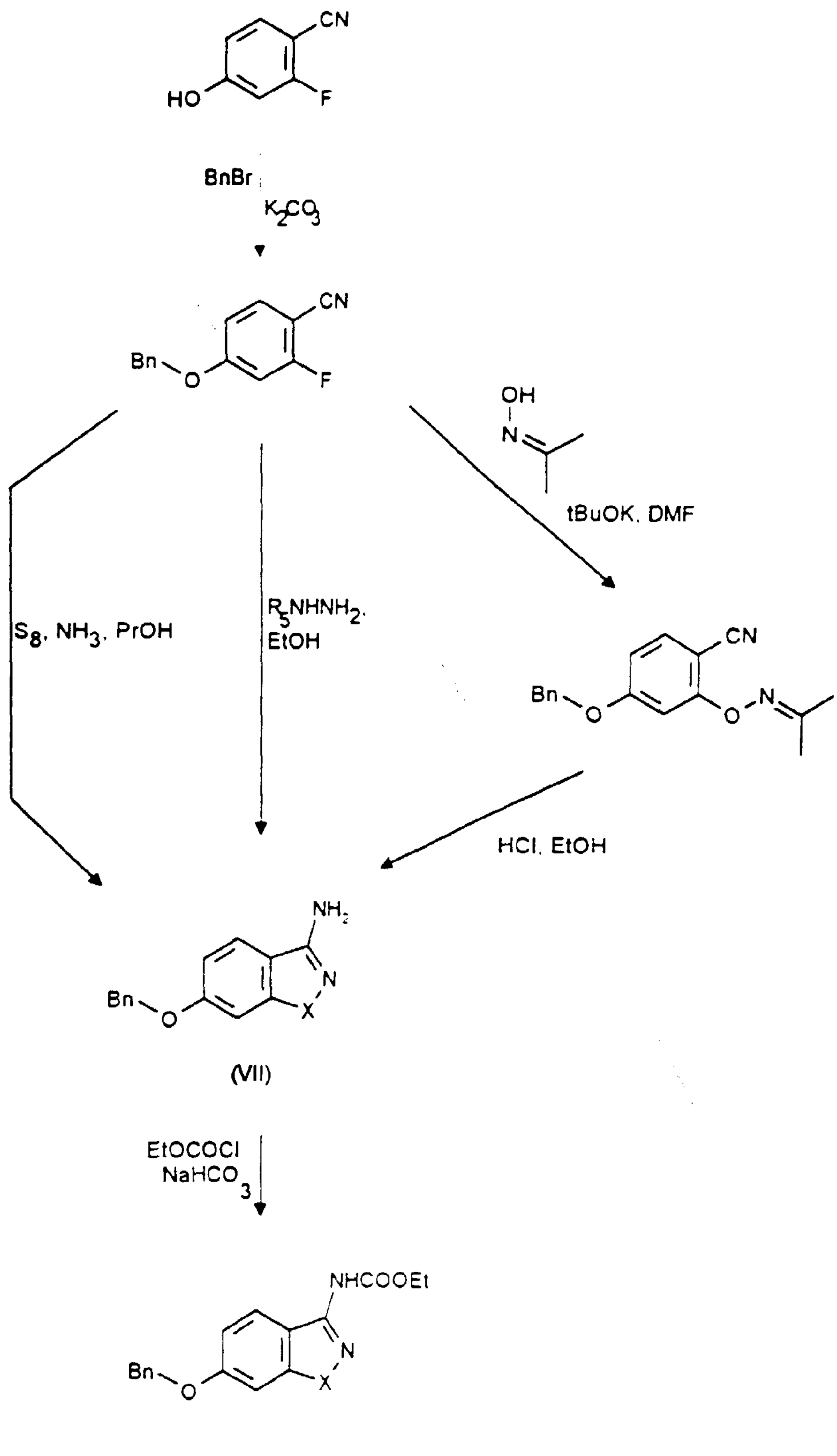
BnBr
$K_2CO_3$
tBuOK, DMF
$S_8$, $NH_3$, PrOH
$R_5NHNH_2$, EtOH
HCl, EtOH
(VII)
EtOCOCl
$NaHCO_3$

(II)

## Revendications

1. Dérivés de 5-(hydroxyméthyl)oxazolidin-2-one de formule générale (I)

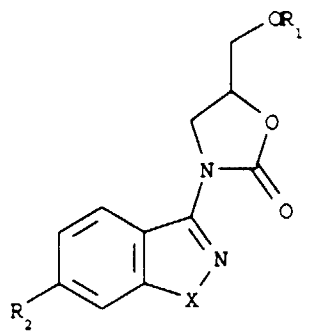

(I)

dans laquelle :

X représente un atome d'oxygène, un atome de soufre, ou un groupe NR, R étant un atome d'hydrogène, ou une chaîne alkyle en $C_1$-$C_4$, linéaire ou ramifiée,
$R_1$ représente un atome d'hydrogène ou un groupe méthyle, et
$R_2$ représente :

(i) un groupe $R_3O$ dans lequel $R_3$ représente soit un atome d'hydrogène, soit un groupe benzyle éventuellement substitué par un atome d'halogène ou par un groupe nitro ou méthylènedioxy, soit un groupe méthoxyéthyle, butyle, 4,4,4-trifluorobutyle, 4,4,4-trifluoro-3-hydroxybutyle ou 4,4,4-trifluorobut-2-ényle, ou (ii) un groupe -CH=CH-$R_4$ ou -$CH_2$-$CH_2$-$R_4$ et dans lequel $R_4$ représente un atome d'hydrogène ou un groupe phényle, 3,3,3-trifluoropropyle ou 3,3,3-trifluoro-2-hydroxypropyle,

sous forme d'énantiomères ou de diastéréoisomères, ou de mélanges de ces différentes formes, y compris de mélanges racémiques.

2. Dérivés de 5-(hydroxyméthyl)oxazolidin-2-one de formule générale (I) selon la revendication 1, caractérisés en ce que :

X représente un atome d'oxygène, un atome de soufre, ou un groupe NR, R étant un atome d'hydrogène, ou une chaîne alkyle en $C_1$-$C_4$, linéaire ou ramifiée,
$R_1$ représente un groupe méthyle ou un atome d'hydrogène, et
$R_2$ représente un groupe $R_3O$ dans lequel $R_3$ représente soit un atome d'hydrogène, soit un groupe benzyle éventuellement substitué par un atome d'halogène ou par un groupe nitro ou méthylènedioxy, soit un groupe méthoxyéthyle, butyle, 4,4,4-trifluorobutyle, 4,4,4-trifluoro-3-hydroxybutyle ou 4,4,4-trifluorobut-2-ényle,

sous forme d'énantiomères ou de diastéréoisomères, ou de mélanges de ces différentes formes, y compris de mélanges racémiques.

3. Dérivés de 5-(hydroxyméthyl)oxazolidin-2-one de formule générale (I) selon la revendication 1, caractérisés en ce que :

X représente un atome d'oxygène, un atome de soufre, ou un groupe NR, R étant un atome d'hydrogène, ou une chaîne alkyle en $C_1$-$C_4$, linéaire ou ramifiée,
$R_1$ représente un groupe méthyle ou un atome d'hydrogène, et
$R_2$ représente un groupe -CH=CH-$R_4$ ou -$CH_2$-$CH_2$-$R_4$ et dans lequel $R_4$ représente un atome d'hydrogène ou un groupe phényle, 3,3,3-trifluoropropyle ou 3,3,3-trifluoro-2-hydroxypropyle,

sous forme d'énantiomères ou de diastéréoisomères, ou de mélanges de ces différentes formes, y compris de mélanges racémiques.

4. Dérivés de 5-(hydroxyméthyl)oxazolidin-2-one de formule générale (I) selon la revendication 1, caractérisés en ce que :

X représente un atome d'oxygène,
$R_1$ représente un groupe méthyle ou un atome d'hydrogène, et
$R_2$ représente :

(i) un groupe $R_3O$ dans lequel $R_3$ représente soit un atome d'hydrogène, soit un groupe benzyle, éventuellement substitué par un atome d'halogène ou par un groupe nitro ou méthylènedioxy, soit un groupe méthoxyéthyle, butyle, 4,4,4-trifluorobutyle, 4,4,4-trifluoro-3-hydroxybutyle ou 4,4,4-trifluorobut-2-ényle, ou (ii) un groupe -CH=CH-$R_4$ ou -$CH_2$-$CH_2$-$R_4$ et dans lequel $R_4$ représente un atome d'hydrogène ou un groupe phényle, 3,3,3-trifluoropropyle ou 3,3,3-trifluoro-2-hydroxypropyle,

sous forme d'énantiomères ou de diastéréoisomères, ou de mélanges de ces différentes formes, y compris de mélanges racémiques.

5. Dérivés de 5-(hydroxyméthyl)oxazolidin-2-one de formule générale (I) selon la revendication 1, caractérisés en ce que :

X représente un atome d'oxygène,
$R_1$ représente un groupe méthyle ou un atome d'hydrogène, et
$R_2$ représente soit un groupe hydroxyle, soit un groupe phénylméthoxyle, éventuellement substitué par un atome d'halogène ou par un groupe nitro ou méthylènedioxy, soit un groupe 4,4,4-trifluorobutoxyle, soit un groupe 4,4,4-trifluoro-3-hydroxybutoxyle,

sous forme d'énantiomères ou de diastéréoisomères, ou de mélanges de ces différentes formes, y compris de mélanges racémiques.

6. Un composé selon la revendication 1, la (*S*)-5-méthoxyméthyl-3-[6-(4,4,4-trifluorobutoxy)-1,2-benzisoxazol-3-yl] oxazolidin-2-one,

7. Procédé de préparation des composés de formule (I) selon la revendication 1 dans laquelle :

X représente un atome d'oxygène, ou un atome de soufre, ou un groupe NR, R étant un atome d'hydrogène, ou une chaîne alkyle en $C_1$-$C_4$, linéaire ou ramifiée,
$R_1$ représente un groupe méthyle,
$R_2$ représente un groupe $R_3O$ dans lequel $R_3$ représente soit un atome d'hydrogène, soit un groupe benzyle, éventuellement substitué par un atome d'halogène ou par un groupe nitro ou méthylènedioxy, soit un groupe méthoxyéthyle, butyle, 4,4,4-trifluorobutyle, 4,4,4-trifluoro-3-hydroxybutyle ou 4,4,4-trifluorobut-2-ényle,

procédé caractérisé en ce que l'on traite le composé de formule (II)

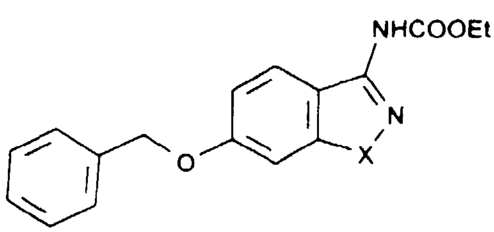

(II)

par l'un des isomères 4(*R*) ou 4(*S*) de la 4-méthoxyméthyl-1,3-dioxolan-2-one de formule (IIIa)

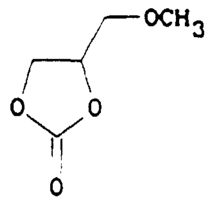

(IIIa)

en présence de carbonate de potassium, pour obtenir l'isomère 5*(S)* ou 5*(R)* du composé de formule (Ia)

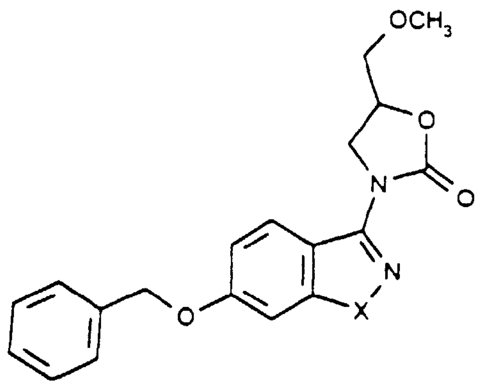

(Ia)

que l'on débenzyle par hydrogénation catalytique, ou à l'aide d'un acide de Lewis, pour obtenir l'isomère 5*(S)* ou 5*(R)* du composé de formule ((Ib), $R_1$=$CH_3$)

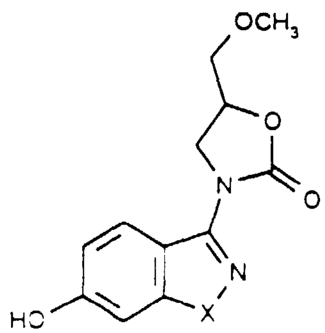

(Ib) $R_1$=$CH_3$

que l'on traite

soit par un composé de formule $R_3Y$ dans laquelle $R_3$ est défini comme dans la formule (I), à l'exception des valeurs hydrogène et benzyle non substitué, et Y est un groupement partant tel qu'un atome de chlore ou de brome ou un groupe tosyloxy, en présence de carbonate de potassium, soit par un composé de formule $R_3OH$ dans laquelle $R_3$ est défini comme précédemment, en présence de triphénylphosphine et d'azodicarboxylate de diéthyle, pour obtenir les isomères 5*(S)* ou 5*(R)* des composés de formule ((Ic) $R_1$=$CH_3$)

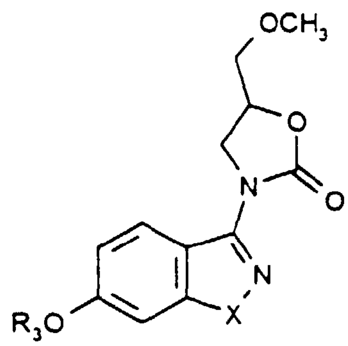

(Ic) $R_1=CH_3$

et, si nécessaire, on traite les composés tels que $X=NCH_3$ par du peroxyde de benzoyle pour obtenir les composés de formule (Ih) dans lesquels X=NH

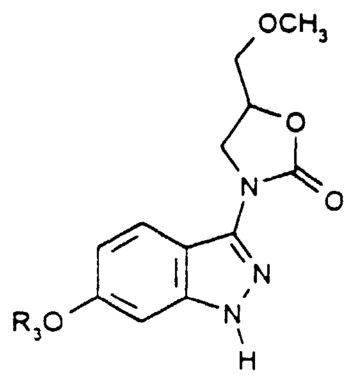

(Ih)

**8.** Procédé de préparation des composés de formule (I) selon la revendication 1 dans laquelle $R_1$ représente un atome d'hydrogène, les substituants X et $R_2$ ayant la même signification que précédemment, caractérisé en ce que l'on traite le 6-phénylméthoxy-1,2-benzisoxazol-3-carbamate d'éthyle de formule (II)

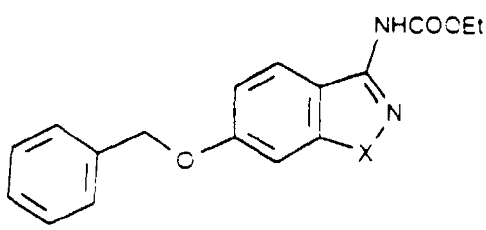

(II)

par l'un des isomères 4*(S)* ou 4*(R)* de la 4-phénylméthoxy méthyl-1,3-dioxolan-2-one de formule (IIIb)

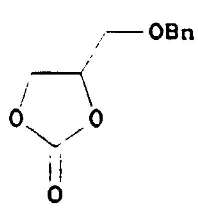

(IIIb)

en présence de carbonate de potassium, pour obtenir l'isomère 5*(R)* ou 5*(S)* du composé de formule (VI)

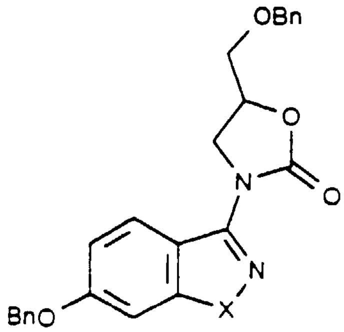

(VI)

que l'on débenzyle par hydrogénation catalytique, pour obtenir l'isomère 5*(R)* ou 5*(S)* du composé de formule ((Ib), $R_1$=H)

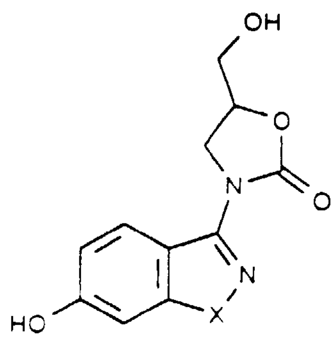

(Ib) $R_1$=H

puis que l'on traite par un composé de formule $R_3$Y dans laquelle $R_3$ est défini comme dans la formule (I), à l'exception de la valeur hydrogène et Y est un groupement partant tel qu'un atome de chlore ou de brome ou un groupe tosyloxy, en présence de carbonate de potassium, pour obtenir les isomères 5*(R)* ou 5*(S)* des composés de formule ((Ic), $R_1$=H)

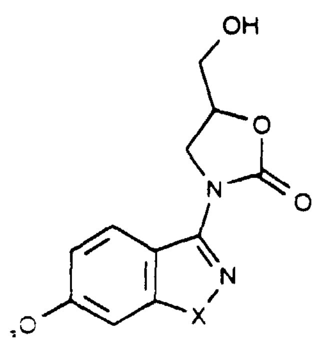

(Ic), $R_1$=H

**9.** Procédé de préparation des composés de formule (I) selon la revendication 1 dans laquelle :

X représente un atome d'oxygène, un atome de soufre, ou un groupe NR, R étant un atome d'hydrogène, ou une chaîne alkyle en $C_1$-$C_4$, linéaire ou ramifiée,
$R_1$ représente un atome d'hydrogène ou un groupe méthyle, et
$R_2$ représente un groupe -CH=CH-$R_4$ ou -$CH_2$-$CH_2$-$R_4$ et dans lequel $R_4$ représente un atome d'hydrogène ou un groupe phényle, 3,3,3-trifluoropropyle ou 3,3,3-trifluoro-2-hydroxypropyle, caractérisé en ce qu'il consiste à traiter le composé de formule ((Ib), $R_1$=$CH_3$)

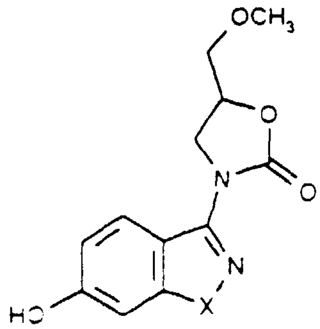

(Ib)

par l'anhydride trifluorométhanesulfonique, à faire réagir le composé de formule (IV) ainsi obtenu

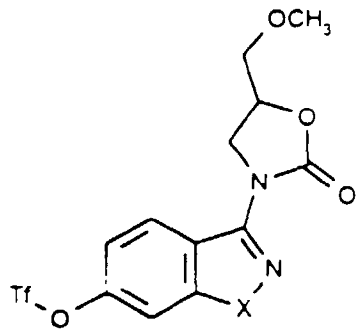

(IV)

avec le tributylvinylétain en présence de chlorure de lithium et de tétrakis (triphénylphosphine)palladium, à traiter le composé de formule (Id)

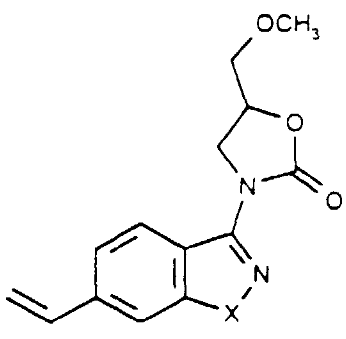

(Id)

par de l'ozone puis par le sulfure de diméthyle, à faire réagir le composé de formule (V)

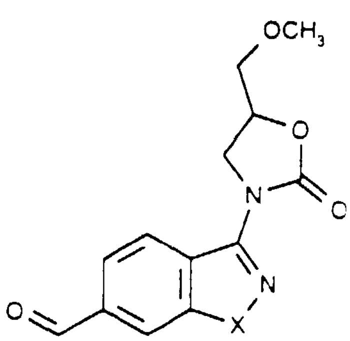

(V)

avec un iodure de triphénylphosphonium de formule $R_4CH_2PPh_3^{+}I^{-}$ dans laquelle $R_4$ est défini comme dans la formule (I) ci-dessus, à l'exception de la valeur hydrogène, en présence de carbonate de potassium, à réduire le composé de formule (Ie)

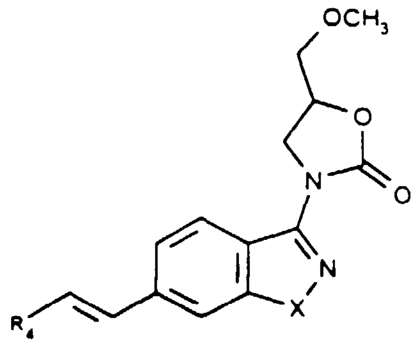

(Ie)

par l'hydrogène en présence d'un catalyseur pour obtenir le composé de formule (If)

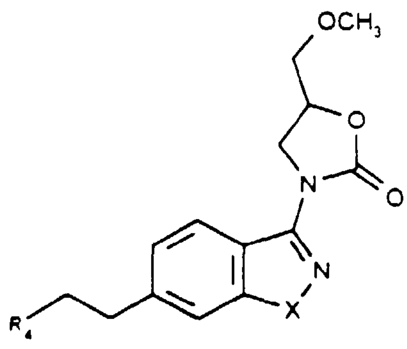

(If)

dans laquelle $R_4$ est défini comme précédemment, et enfin à traiter ce composé par du tribromure de bore, pour obtenir le composé de formule (Ig)

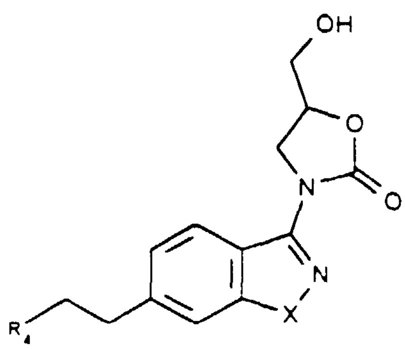

(Ig)

**10.** Médicament caractérisé en ce qu'il est constitué par un composé de formule (I) selon la revendication 1.

**11.** Composition pharmaceutique caractérisée en ce qu'elle comprend un composé de formule (I) selon la revendication 1 en association avec tout excipient approprié.

**Patentansprüche**

**1.** 5-(Hydroxymethyl)-oxazolidin-2-on-Derivate der allgemeinen Formel (1)

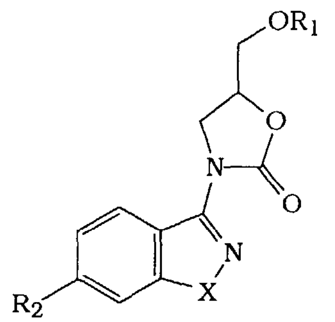

(I)

in der:

X ein Sauerstoffatom, ein Schwefelatom oder eine Gruppe NR, worin R ein Wasserstoffatom oder eine geradkettige oder verzweigte $C_1$-$C_4$-Alkylkette darstellt,
$R_1$ ein Wasserstoffatom oder eine Methylgruppe und
$R_2$:

(i) eine Gruppe $R_3O$, worin $R_3$ entweder ein Wasserstoffatom oder eine gegebenenfalls durch ein Halogenatom oder eine Nitrogruppe oder eine Methylendioxygruppe substituierte Benzylgruppe, oder eine Methoxyethylgruppe, eine Butylgruppe, eine 4,4,4-Trifluorbutylgruppe, eine 4,4,4-Trifluor-3-hydroxybutylgruppe oder eine 4,4,4-Trifluorbut-2-enyl-gruppe darstellt, oder
(ii) eine Gruppe -CH=CH-$R_4$ oder -$CH_2$-$CH_2$-$R_4$, worin $R_4$ ein Wasserstoffatom oder eine Phenylgruppe, eine 3,3,3-Trifluorpropylgruppe oder eine 3,3,3-Trifluor-2-hydroxypropylgruppe darstellt,

bedeuten, in Form der Enantiomeren oder der Diastereoisomeren oder in Form von Mischungen dieser verschiedenen Formen, einschließlich racemischer Mischungen.

**2.** 5-(Hydroxymethyl)-oxazolidin-2-on-Derivate der allgemeinen Formel (I) nach Anspruch 1, dadurch gekennzeichnet, daß:

X ein Sauerstoffatom, ein Schwefelatom oder eine Gruppe NR, worin R ein Wasserstoffatom oder eine geradkettige oder verzweigte $C_1$-$C_4$-Alkylkette darstellt,
$R_1$ eine Methylgruppe oder ein Wasserstoffatom und
$R_2$ eine Gruppe $R_3O$, worin $R_3$ entweder ein Wasserstoffatom oder eine gegebenenfalls durch ein Halogenatom oder eine Nitrogruppe oder eine Methylendioxygruppe substituierte Benzylgruppe, oder eine Methoxyethylgruppe, eine Butylgruppe, eine 4,4,4-Trifluorbutylgruppe, eine 4,4,4-Trifluor-3-hydroxybutylgruppe oder eine 4,4,4-Trifluorbut-2-enyl-gruppe darstellt, bedeuten,

in Form der Enantiomeren oder der Diastereoisomeren oder von Mischungen dieser verschiedenen Formen einschließlich racemischer Mischungen.

**3.** 5-(Hydroxymethyl)-oxazolidin-2-on-Derivate der allgemeinen Formel (I) nach Anspruch 1, dadurch gekennzeichnet, daß:

X ein Sauerstoffatom, ein Schwefelatom oder eine Gruppe NR, worin R ein Wasserstoffatom oder eine geradkettige oder verzweigte $C_1$-$C_4$-Alkylkette darstellt,
$R_1$ eine Methylgruppe oder ein Wasserstoffatom und
$R_2$ eine Gruppe -CH=CH-$R_4$ oder -$CH_2$-$CH_2$-$R_4$, worin $R_4$ ein Wasserstoffatom oder eine Phenylgruppe, eine 3,3,3-Trifluorpropylgruppe oder eine 3,3,3-Trifluor-2-hydroxypropylgruppe darstellt, bedeuten,

in Form der Enantiomeren oder der Diastereoisomeren oder von Mischungen dieser verschiedenen Formen einschließlich racemischer Mischungen.

**4.** 5-(Hydroxymethyl)-oxazolidin-2-on-Derivate der allgemeinen Formel (I) nach Anspruch 1, dadurch gekennzeichnet, daß:

X ein Sauerstoffatom,
$R_1$ eine Methylgruppe oder ein Wasserstoffatom und
$R_2$:

(i) eine Gruppe $R_3O$, worin $R_3$ entweder eine gegebenenfalls durch ein Halogenatom oder eine Nitrogruppe oder eine Methylendioxygruppe substituierte Benzylgruppe, oder eine Methoxyethylgruppe, eine Butylgruppe, eine 4,4,4-Trifluorbutylgruppe, eine 4,4,4-Trifluor-3-hydroxybutylgruppe oder eine 4,4,4-Trifluorbut-2-enyl-gruppe darstellt, oder
(ii) eine Gruppe $-CH{=}CH-R_4$ oder $-CH_2-CH_2-R_4$, worin $R_4$ ein Wasserstoffatom oder eine Phenylgruppe, eine 3,3,3-Trifluorpropylgruppe oder eine 3,3,3-Trifluor-2-hydroxypropylgruppe darstellt,

in Form der Enantiomeren oder der Diastereoisomeren oder von Mischungen dieser verschiedenen Formen einschließlich racemischer Mischungen.

**5.** 5-(Hydroxymethyl)-oxazolidin-2-on-Derivate der allgemeinen Formel (I) nach Anspruch 1, dadurch gekennzeichnet, daß:

X ein Sauerstoffatom,
$R_1$ eine Methylgruppe oder ein Wasserstoffatom und
$R_2$ entweder eine Hydroxygruppe oder eine gegebenenfalls durch ein Halogenatom oder eine Nitrogruppe oder eine Methylendioxygruppe substituierte Phenylmethoxygruppe oder eine 4,4,4-Trifluorbutoxygruppe oder eine 4,4,4-Trifluor-3-hydroxybutoxygruppe darstellt, bedeuten,

in Form der Enantiomeren oder der Diastereoisomeren oder von Mischungen dieser verschiedenen Formen einschließlich racemischer Mischungen.

**6.** Verbindung nach Anspruch 1, nämlich *(S)*-5 -Methoxymethyl-3-[6-(4,4,4-trifluorbutoxy)-1,2-benzisoxazol-3-yl]-oxazolidin-2-on.

**7.** Verfahren zur Herstellung der Verbindungen der Formel (I) nach Anspruch 1, worin:

X ein Sauerstoffatom oder ein Schwefelatom oder eine Gruppe NR, worin R ein Wasserstoffatom oder eine geradkettige oder verzweigte $C_1$-$C_4$-Alkylkette darstellt,
$R_1$ eine Methylgruppe und
$R_2$ eine Gruppe $R_3O$, worin $R_3$ entweder ein Wasserstoffatom oder eine gegebenenfalls durch ein Halogenatom oder eine Nitrogruppe oder eine Methylendioxygruppe substituierte Benzylgruppe, oder eine Methoxyethylgruppe, eine Butylgruppe, eine 4,4,4-Trifluorbutylgruppe, eine 4,4,4-Trifluor-3-hydroxybutylgruppe oder eine 4,4,4-Trifluorbut-2-enyl-gruppe darstellt, bedeuten,

dadurch gekennzeichnet, daß man die Verbindung der Formel (II)

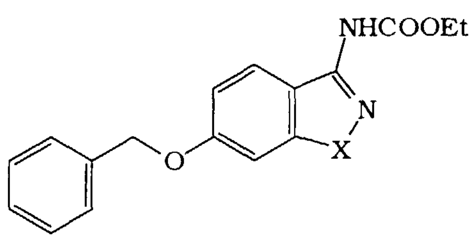

(II)

mit einem der 4*(R)*- oder 4-*(S)*-Isomeren von 4-Methoxymethyl-1,3-dioxolan-2-on der Formel (IIIa)

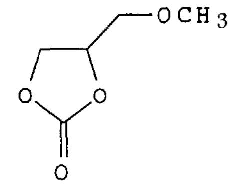

(IIIa)

in Gegenwart von Kaliumcarbonat umsetzt zur Bildung des 5-*(S)*- oder 5-(*R*)-Isomeren der Verbindung der Formel (Ia)

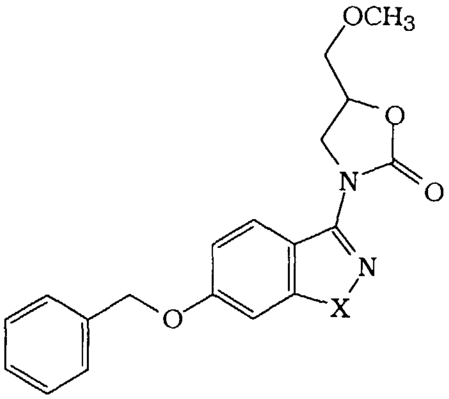

(Ia)

welche man durch katalytische Hydrierung oder mit Hilfe einer Lewis-Säure debenzyliert zur Bildung des 5-*(S)*- oder 5-*(R)*-Isomeren der Verbindung der Formel ((Ib), $R_1 = CH_3$)

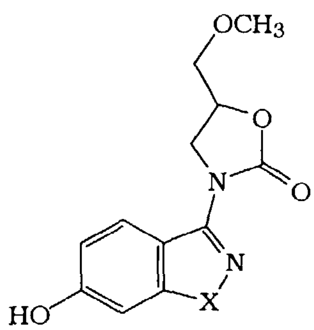

(Ib) $R_1 = CH_3$

welche man entweder

in Gegenwart von Kaliumcarbonat mit einer Verbindung der Formel $R_3Y$, in der $R_3$ die bezüglich der Formel (I) angegebenen Bedeutungen mit Ausnahme von Wasserstoff und nichtsubstituiertem Benzyl besitzt und Y eine austretende Gruppe, wie ein Chloratom oder ein Bromatom oder eine Tosyloxygruppe bedeutet,

oder in Gegenwart von Triphenylphosphin und Azodicarbonsäurediethylester mit einer Verbindung der Formel $R_3OH$, worin $R_3$ die oben angegebenen Bedeutungen besitzt, umsetzt zur Bildung der 5-*(S)*- oder 5-(*R*)-Isomeren der Verbindungen der Formel ((Ic), $R_1 = CH_3$)

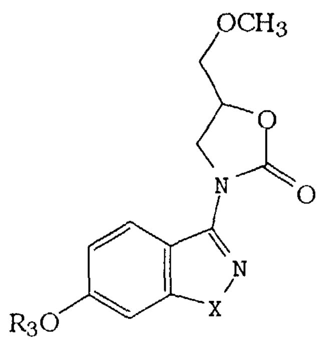

(Ic) $R_1 = CH_3$

und erforderlichenfalls die Verbindungen, worin X = $NCH_3$ darstellt, mit Benzoylperoxid behandelt zur Bildung der Verbindungen der Formel (Ih)

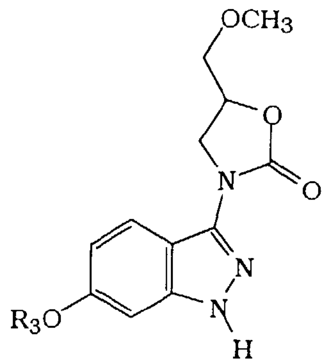

(Ih)

in der X = NH bedeutet.

**8.** Verfahren zur Herstellung der Verbindungen der Formel (I) nach Anspruch 1, worin $R_1$ ein Wasserstoffatom darstellt und die Substituenten X und $R_2$ die oben angegebenen Bedeutungen besitzen, dadurch gekennzeichnet, daß man 6-Phenylmethoxy-1,2-benzisoxazol-3-carbamidsäureethylester der Formel (II)

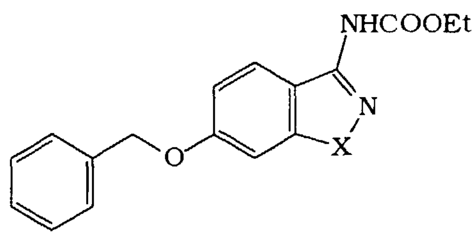

(II)

mit einem der 4-*(S)*- oder 4-*(R)*-Isomeren von 4-Phenylmethoxymethyl-1,3-dioxolan-2-on der Formel (IIIb)

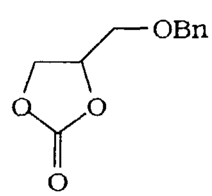

(IIIb)

in Gegenwart von Kaliumcarbonat behandelt zur Bildung des 5-*(R)*- oder 5-*(S)*-Isomeren der Verbindung der Formel (VI)

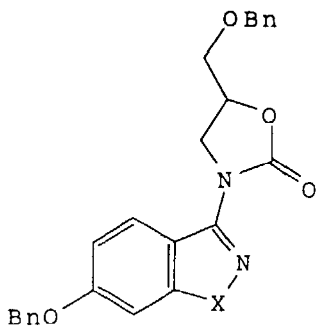

(VI)

welche man durch katalytische Hydrierung debenzyliert zur Bildung des 5-(*R*)- oder 5-(S)-Isomeren der Verbindung der Formel ((Ib), $R_1$ = H)

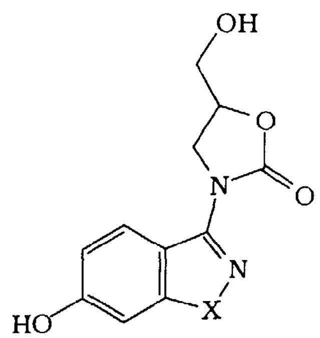

(Ib) $R_1$ = H

welches man in Gegenwart von Kaliumcarbonat mit einer Verbindung der Formel $R_3Y$, worin $R_3$ die bezüglich der Formel (I) angegebenen Bedeutungen besitzt mit Ausnahme von Wasserstoff, und Y eine austretende Gruppe, wie ein Chloratom oder ein Bromatom oder eine Tosyloxygruppe darstellt, behandelt zur Bildung der 5-(*R*)-oder 5-*(S)*-Isomeren der Verbindungen der Formel ((Ic), $R_1$ = H)

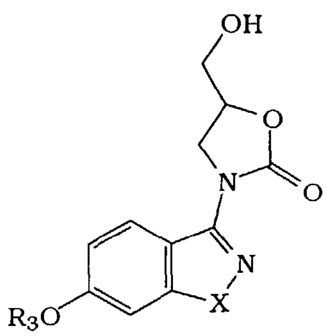

(Ic) $R_1$ = H

**9.** Verfahren zur Herstellung der Verbindungen der Formel (I) gemäß Anspruch 1, in der:

X ein Sauerstoffatom, ein Schwefelatom oder eine Gruppe NR, worin R ein Wasserstoffatom oder eine geradkettige oder verzweigte $C_1$-$C_4$-Alkylkette darstellt,
$R_1$ ein Wasserstoffatom oder eine Methylgruppe und
$R_2$ eine Gruppe -CH=CH-$R_4$ oder -$CH_2$-$CH_2$-$R_4$, worin $R_4$ ein Wasserstoffatom oder eine Phenylgruppe, eine 3,3,3-Trifluorpropylgruppe oder eine 3,3,3-Trifluor-2-hydroxypropylgruppe darstellt, bedeuten, dadurch gekennzeichnet, daß man die Verbindung der Formel ((Ib), $R_1$ = $CH_3$)

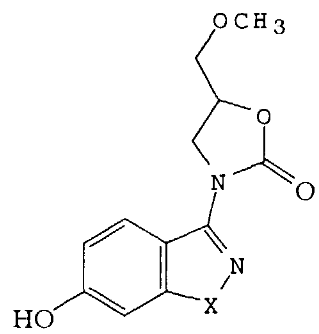

(Ib)

mit Trifluormethansulfonsäureanhydrid behandelt, die in dieser Weise erhaltene Verbindung der Formel (IV)

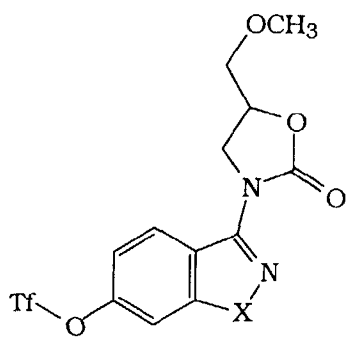

(IV)

in Gegenwart von Lithiumchlorid und Tetrakis(triphenylphosphin)-palladium mit Tributylvinylzinn umsetzt, die Verbindung der Formel (Id)

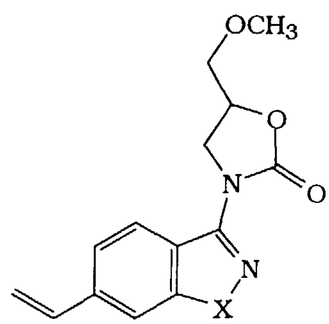

(Id)

mit Ozon und dann mit Dimethylsulfid behandelt, die Verbindung der Formel (V)

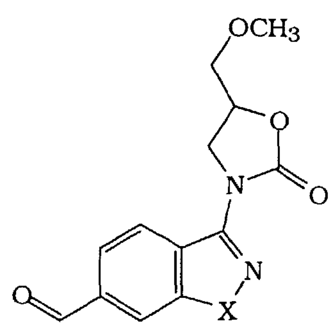

(V)

mit einem Triphenylphosphoniumiodid der Formel $R_4CH_2PPh_3{}^+I^-$, worin $R_4$ die oben bezüglich der Formel (I)

angegebenen Bedeutungen mit Ausnahme von Wasserstoff besitzt, in Gegenwart von Kaliumcarbonat behandelt, die Verbindung der Formel (Ie)

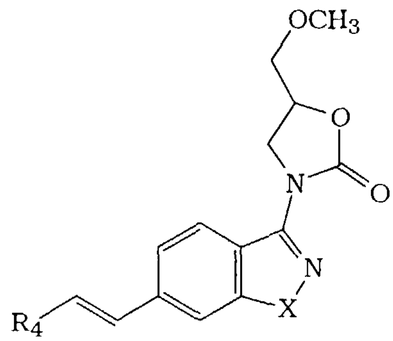

(Ie)

mit Wasserstoff in Gegenwart eines Katalysators reduziert zur Bildung der Verbindung der Formel (If)

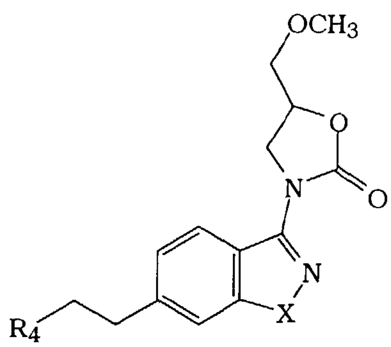

(If)

in der $R_4$ die oben angegebenen Bedeutungen besitzt, und schließlich diese Verbindung mit Bortribromid behandelt zur Bildung der Verbindung der Formel (Ig)

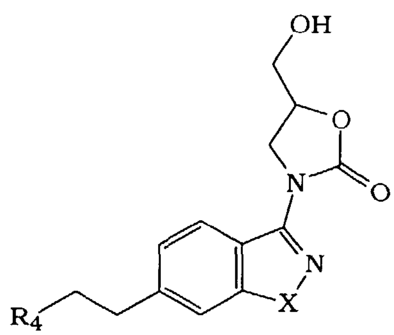

(Ig)

**10.** Arzneimittel, dadurch gekennzeichnet, daß es aus einer Verbindung der Formel (I) nach Anspruch 1 gebildet ist.

**11.** Pharmazeutische Zubereitung, dadurch gekennzeichnet, daß sie eine Verbindung der Formel (I) nach Anspruch 1 in Kombination mit einem beliebigen geeigneten Trägermaterial umfaßt.

**Claims**

**1.** 5-(Hydroxymethyl)oxazolidin-2-one derivatives of general formula (I)

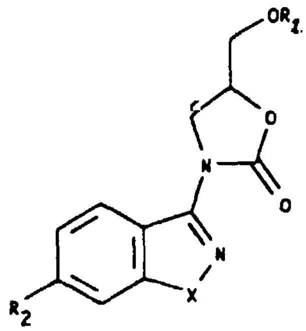

(I)

in which:

X represents an oxygen atom, a sulphur atom or a group NR, R being a hydrogen atom or a linear or branched $C_1$-$C_4$ alkyl chain,
$R_1$ represents a hydrogen atom or a methyl group, and
$R_2$ represents:

(i) a group $R_3O$ in which $R_3$ represents alternatively a hydrogen atom, or a benzyl group which is optionally substituted with a halogen atom or with a nitro or methylenedioxy group, or represents a methoxyethyl, butyl, 4,4,4-trifluorobutyl, 4,4,4-trifluoro-3-hydroxybutyl or 4,4,4-trifluorobut-2-enyl group,
or (ii) a group -CH=CH-$R_4$ or -$CH_2$-$CH_2$-$R_4$, in which $R_4$ represents a hydrogen atom or a phenyl, 3,3,3-trifluoropropyl or 3,3,3-trifluoro-2-hydroxypropyl group,

in the form of enantiomers or diastereoisomers, or of mixtures of these various forms, including racemic mixtures.

**2.** 5-(Hydroxymethyl)oxazolidin-2-one derivatives of the general formula (I) according to Claim 1, characterized in that:

X represents an oxygen atom, a sulphur atom or a group NR, R being a hydrogen atom or a linear or branched $C_1$-$C_4$ alkyl chain,
$R_1$ represents a methyl group or a hydrogen atom, and
$R_2$ represents a group $R_3O$ in which $R_3$ represents alternatively a hydrogen atom, or a benzyl group which is optionally substituted with a halogen atom or with a nitro or methylenedioxy group, or represents a methoxyethyl, butyl, 4,4,4-trifluorobutyl, 4,4,4-trifluoro-3-hydroxybutyl or 4,4,4-trifluorobut-2-enyl group,

in the form of enantiomers or diastereoisomers, or of mixtures of these various forms, including racemic mixtures.

**3.** 5-(Hydroxymethyl)oxazolidin-2-one derivatives of the general formula (I) according to Claim 1, characterized in that:

X represents an oxygen atom, a sulphur atom or a group NR, R being a hydrogen atom or a linear or branched $C_1$-$C_4$ alkyl chain,
$R_1$ represents a methyl group or a hydrogen atom, and
$R_2$ represents a group -CH=CH-$R_4$ or -$CH_2$-$CH_2$-$R_4$, in which $R_4$ represents a hydrogen atom, or a phenyl, 3,3,3-trifluoropropyl or 3,3,3-trifluoro-2-hydroxypropyl group,

in the form of enantiomers or diastereoisomers, or of mixtures of these various forms, including racemic mixtures.

**4.** 5-(Hydroxymethyl)oxazolidin-2-one derivatives of the general formula (I) according to Claim 1, characterized in that:

X represents an oxygen atom,
$R_1$ represents a methyl group or a hydrogen atom, and

$R_2$ represents:

(i) a group $R_3O$ in which $R_3$ represents alternatively a hydrogen atom, or a benzyl group which is optionally substituted with a halogen atom or with a nitro or methylenedioxy group, or represents a methoxyethyl, butyl, 4,4,4-trifluorobutyl, 4,4,4-trifluoro-3-hydroxybutyl or 4,4,4-trifluorobut-2-enyl group,
or (ii) a group -CH=CH-$R_4$ or -$CH_2$-$CH_2$-$R_4$, in which $R_4$ represents a hydrogen atom, or a phenyl, 3,3,3-trifluoropropyl or 3,3,3-trifluoro-2-hydroxypropyl group,

in the form of enantiomers or diastereoisomers, or of mixtures of these various forms, including racemic mixtures.

**5.** 5-(Hydroxymethyl)oxazolidin-2-one derivatives of the general formula (I) according to Claim 1, characterized in that:

X represents an oxygen atom,
$R_1$ represents a methyl group or a hydrogen atom, and
$R_2$ represents alternatively a hydroxyl group, or a phenylmethoxy group which is optionally substituted with a halogen atom or with a nitro or methylenedioxy group, or represents a 4,4,4-trifluorobutoxy group or a 4,4,4-trifluoro-3-hydroxybutoxy group,

in the form of enantiomers or diastereoisomers, or of mixtures of these various forms, including racemic mixtures.

**6.** A compound according to Claim 1, (S)-5-methoxymethyl-3-[6-(4,4,4-trifluorobutoxy)-1,2-benzisoxazol-3-yl]oxazolidin-2-one.

**7.** Process for the preparation of compounds of formula (I) according to Claim 1, in which:

X represents an oxygen atom, a sulphur atom or a group NR, R being a hydrogen atom or a linear or branched $C_1$-$C_4$ alkyl chain,
$R_1$ represents a methyl group, and
$R_2$ represents a group $R_3O$ in which $R_3$ represents alternatively a hydrogen atom, or a benzyl group which is optionally substituted with a halogen atom or with a nitro or methylenedioxy group, or represents a methoxyethyl, butyl, 4,4,4-trifluorobutyl, 4,4,4-trifluoro-3-hydroxybutyl or 4,4,4-trifluorobut-2-enyl group,

characterized in that the compound of formula (II)

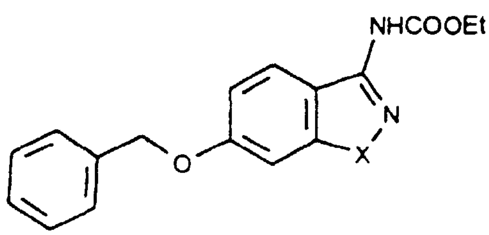

(II)

is treated with one of the 4(R) or 4(S) isomers of 4-methoxymethyl-1,3-dioxolan-2-one of formula (IIIa)

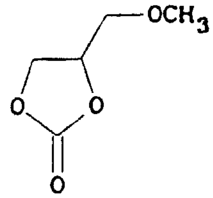

(IIIa)

in the presence of potassium carbonate in order to obtain the 5(S) or 5(R) isomer of the compound of formula (Ia)

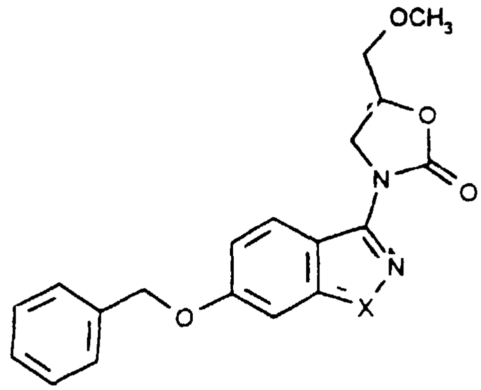

(Ia)

which is debenzylated by catalytic hydrogenation or with the aid of a Lewis acid in order to obtain the 5(S) or 5(R) isomer of the compound of formula ((Ib), $R_1=CH_3$)

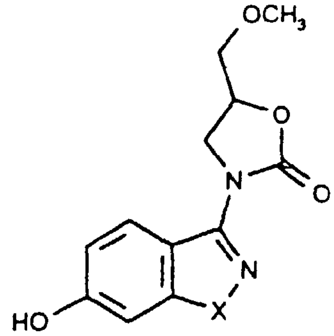

(Ib) $R_1=CH_3$

which is treated

either with a compound of formula $R_3Y$ in which $R_3$ is defined as in formula (I), with the exception of the meanings hydrogen and unsubstituted benzyl, and Y is a leaving group such as a chlorine or bromine atom or a tosyloxy group, in the presence of potassium carbonate,

or with a compound of formula $R_3OH$ in which $R_3$ is defined as above, in the presence of triphenylphosphine and diethyl azodicarboxylate, in order to obtain the 5(S) or 5(R) isomers of the compounds of formula ((Ic) $R_1=CH_3$)

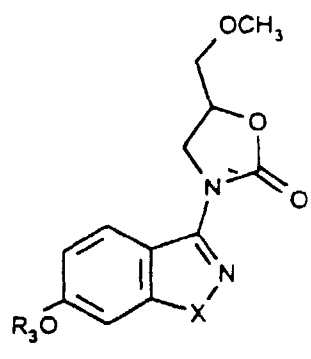

(Ic) $R_1=CH_3$

and, if necessary, the compounds in which $X=NCH_3$ are treated with benzoyl peroxide in order to obtain the compounds of formula (Ih) in which X=NH

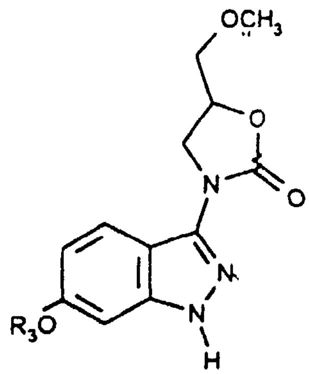

(Ih) .

8. Process for the preparation of compounds of formula (I) according to Claim 1, in which $R_1$ represents a hydrogen atom, the substituents X and $R_2$ having the same meaning as above, characterized in that ethyl 6-phenylmethoxy-1,2-benzisoxazole-3-carbamate of formula (II)

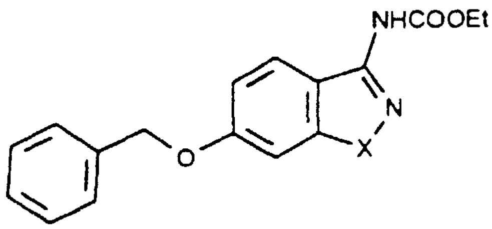

(II)

is treated with one of the 4(S) or 4(R) isomers of 4-phenylmethoxymethyl-1,3-dioxolan-2-one of formula (IIIb)

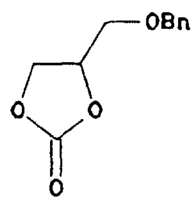

(IIIb)

in the presence of potassium carbonate in order to obtain the 5(R) or 5(S) isomer of the compound of formula (VI)

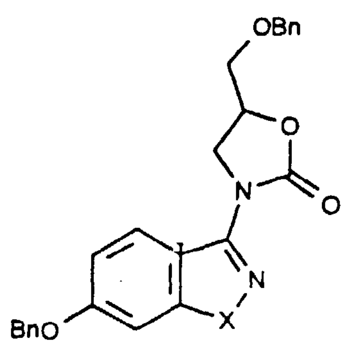

(VI)

which is debenzylated by catalytic hydrogenation in order to obtain the 5(R) or 5(S) isomer of the compound of formula ((Ib), $R_1$=H)

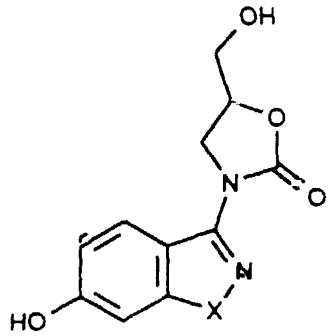

(Ib) $R_1$=H

which is then treated with a compound of formula $R_3Y$ in which $R_3$ is defined as in formula (I), with the exception of the meaning hydrogen, and Y is a leaving group such as a chlorine or bromine atom or a tosyloxy group, in the presence of potassium carbonate, in order to obtain the 5(R) or 5(S) isomers of the compounds of formula ((Ic), $R_1$=H)

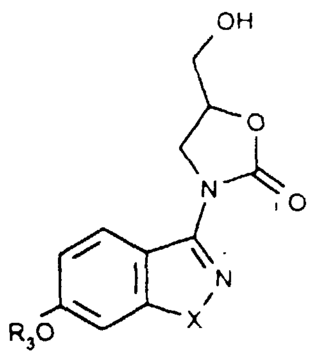

(Ic), $R_1$=H.

**9.** Process for the preparation of compounds of formula (I) according to Claim 1, in which:

X represents an oxygen atom, a sulphur atom or a group NR, R being a hydrogen atom or a linear or branched $C_1$-$C_4$ alkyl chain,
$R_1$ represents a hydrogen atom or a methyl group, and
$R_2$ represents a group -CH=CH-$R_4$ or -$CH_2$-$CH_2$-$R_4$, in which $R_4$ represents a hydrogen atom, or a phenyl, 3,3,3-trifluoropropyl or 3,3,3-trifluoro-2-hydroxypropyl group,

characterized in that it consists in treating the compound of formula ((Ib), $R_1$=$CH_3$)

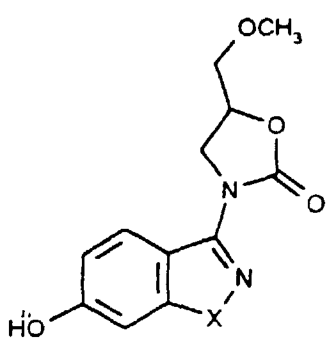

(Ib)

with trifluoromethanesulphonic anhydride, in reacting the resulting compound of formula (IV)

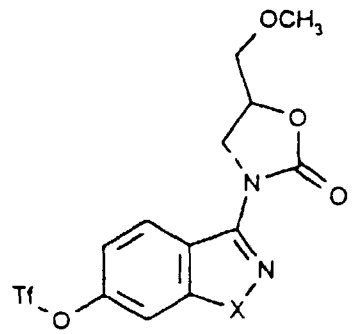

(IV)

with tributylvinyl tin in the presence of lithium chloride and tetrakis(triphenylphosphine)palladium, in treating the compound of formula (Id)

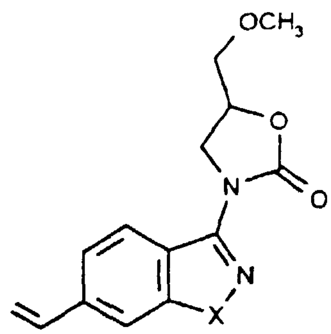

(Id)

with ozone and then with dimethyl sulphide, in reacting the compound of formula (V)

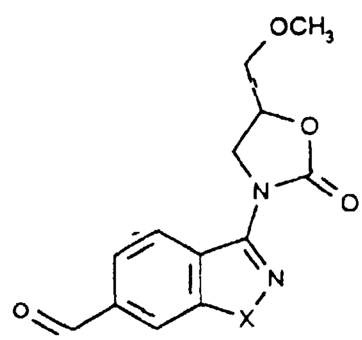

(V)

with a triphenylphosphonium iodide of formula $R_4CH_2PPh_3{}^+I^-$ in which $R_4$ is defined as in formula (I) above, with the exception of the meaning hydrogen, in the presence of potassium carbonate, in reducing the compound of formula (Ie)

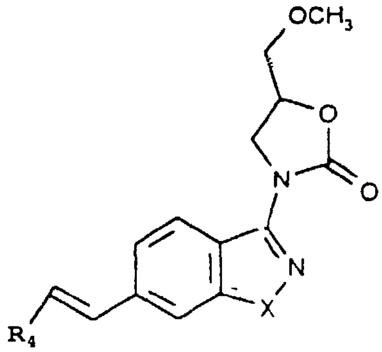

(Ie)

with hydrogen in the presence of a catalyst in order to obtain the compound of formula (If)

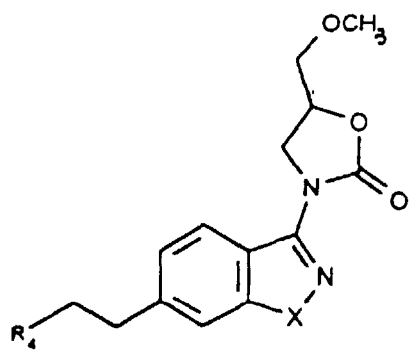

(If)

in which $R_4$ is defined as above, and finally in treating this compound with boron tribromide in order to obtain the compound of formula (Ig)

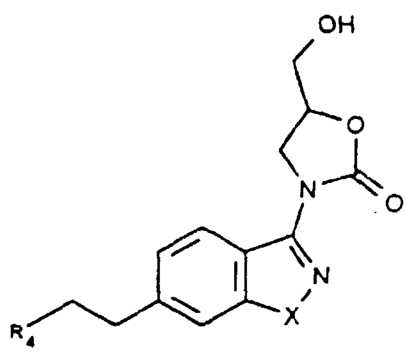

(Ig) .

**10.** Drug, characterized in that it is constituted by a compound of formula (I) according to Claim 1.

**11.** Pharmaceutical composition, characterized in that it comprises a compound of formula (I) according to Claim 1 in combination with any appropriate excipient.